(19) Europäisches Patentamt / European Patent Office / Office européen des brevets

(11) **EP 4 308 627 B1**

(12) **EUROPEAN PATENT SPECIFICATION**

(45) Date of publication and mention
of the grant of the patent:
**07.05.2025 Bulletin 2025/19**

(21) Application number: **21811075.7**

(22) Date of filing: **19.11.2021**

(51) International Patent Classification (IPC):
***C08G 63/183*** (2006.01)   ***G01N 33/44*** (2006.01)
***G01R 33/00*** (2006.01)

(52) Cooperative Patent Classification (CPC):
**C08G 63/183; G01N 33/442; Y02W 30/62**

(86) International application number:
**PCT/EP2021/082384**

(87) International publication number:
**WO 2022/194408 (22.09.2022 Gazette 2022/38)**

(54) **METHOD FOR THE DETERMINATION OF RECYCLED POLYETHYLENE TEREPHTHALATE**

VERFAHREN ZUR BESTIMMUNG VON RECYCELTEM POLYETHYLENTEREPHTHALAT

PROCÉDÉ DE DÉTERMINATION DE TÉRÉPHTALATE DE POLYÉTHYLÈNE RECYCLÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.03.2021 EP 21163268
05.05.2021 EP 21172295**

(43) Date of publication of application:
**24.01.2024 Bulletin 2024/04**

(73) Proprietor: **Worms Safety Europe
1331 Luxembourg (LU)**

(72) Inventors:
• **MULLER DE MOROGUES, Gonzalve
1331 Luxembourg (LU)**
• **LEGOUPIL, Samuel
1331 Luxembourg (LU)**

(74) Representative: **Gevers Patents
De Kleetlaan 7A
1831 Diegem (BE)**

(56) References cited:
**US-A1- 2014 278 142     US-B2- 8 063 374**

**Description**

FIELD OF THE INVENTION

The present invention concerns a method for determining a percentage of recycled polyethylene terephthalate [r-PET, hereinafter] in a material [material M, hereinafter] with regards to the total amount of r-PET and non-recycled polyethylene terephthalate comprised in said material M, said r-PET being derived from at least one polymeric material comprising at least one PET polymer and optionally IPA monomers and optionally TPA monomers wherein said at least one PET polymer is derived from recurring units derived from ethylene glycol and recurring units derived from terephthalic acid [TPA, hereinafter] and recurring units derived from isophthalic acid [IPA, hereinafter];

BACKGROUND OF THE INVENTION

Economic and population growth and global industrialization together cause an increase in the amount of plastic waste. As a consequence of all these factors, while natural resources become increasingly scarce, the plastic wastes created by the ever-increasing consumption tendency have reached the huge amounts that threaten the environment and human health due to their quantities and their harmful and often biologically non-degradable contents.

An example of such a plastic waste material is polyethylene terephthalate (PET). PET is widely used to produce beverage bottles, acoustic panels, packaging trays, blisters, multilayered packaging films, strapping tapes, and various nonpackaging applications such as clothing and textiles, owing to its transparent colour, low specific weight, strength, food safety, fairly inexpensive price, recyclability, etc. In addition to all these advantages, however, PET waste material takes up considerable space. In this regard, the recycling of PET enables conserving precious raw materials, reducing fossil fuel and energy use when compared to the production of virgin PET, and further thereby significantly reducing carbon footprints and greenhouse gas emissions. In this view, nowadays, PET is the most widely recycled plastic in the world.

Bottle grade PET is generally used for water and beverage packaging due to its low specific weight, fairly inexpensive price, resistance to microorganisms, and resistance to light. Consequently, bottles of water, soft drinks, and other beverages constitute a significant part of global PET resin requirement. Because PET absorbs only limited amounts of molecular contaminants during use, the recycling of especially PET bottles has developed greatly over the last years. In 2017 for example, 6.2 M Tonnes of postconsumer PET bottles were effectively recycled in Europe.

With regards to the recycling of PET bottles, said bottles are usually first shredded and chopped into small fragments in order to obtain dirty PET flakes. Then, these dirty PET flakes pass through a series of sorting and cleaning stages to separate PET from other materials that may be contained on the initial PET bottles and/or from any contaminants that might be present (e.g. food residues, glue, paper, aluminium metal).

Most of these recycled PET flakes, as eco-friendly products obtained by recycling of PET bottles, are commonly utilized for fiber applications in the textile and clothing industry. In other words, today, the textile market can offer textiles made of recycled fibers, mainly recycled PET fibers as produced from PET bottles.

Recycled PET fibers are produced by extrusion processes, such as melt spinning processes, of the PET flakes as obtained via the recycling of PET bottles. Often, these recycled PET fibers are mixed up with virgin PET in the production of yarns, woven and non-woven fabrics, and finished textile products. However, it is known that variations of the recycled PET content in PET fibers can potentially have certain drawbacks when used for textile production. Consequently, studies on the use of recycled PET fibers in the field of textile applications have established the focus of researchers worldwide. For example, Yüksekkaya et al. Journal of Engineered Fibers and Fabrics 2016, 11, pages 68-76, investigated the properties of yarns and knitted fabrics produced by virgin PET, recycled PET, and cotton fibers. The authors concluded on the different material properties of virgin PET and recycled PET, respectively, towards the overall product quality of textile products made from said materials. Further, as confirmed in multiple other studies such as disclosed by Telli. et al. Journal of Engineered Fibers and Fabrics 2015, 10, pages 47-60, and by Mari et al. Journal of Textile Engineering 2004, 50, pages 25-30, it remains important, when producing PET textile products comprising recycled PET, to have a clear view on the relative amount of recycled PET material present in the final PET textile products in order to minimize the risk of chemical non-compliance and performance failures while still achieving an optimum quality of said final textile products.

Another issue is that, due to market demands, recycled PET actually costs more than unrecycled PET, it is thus desirable for customers to be able to determine if they pay for the right amount of recycled PET.

For example, US 8,063,374 B2 discloses methods for analyzing plastic materials for determining recycled content in a thermoplastic sample such as PET. The described methodology comprises identifying a reference standard library for thermoplastic content thereby further including the preparation of a plurality of samples of known ratios of virgin thermoplastic versus recycled thermoplastic followed by the analysis of said samples by various techniques such as differential scanning calorimetry, ultraviolet-visible spectroscopy, attenuated total reflectance Fourier transform infrared spectroscopy, or plasma atomic emission spectroscopy.

Unfortunately, current assay techniques are not yet optimal for the easy, rapid, versatile, and reproducible determination of a percentage of recycled polyethylene terephthalate in a material with regards to the total amount of recycled PET and non-recycled PET comprised in said material.

Therefore, in view of all the above, there remains a continuous need for an improved method for determining a percentage of recycled polyethylene terephthalate in a material with regards to the total amount of recycled PET and non-recycled PET comprised in said material, said method being rapid, versatile and reproducible.

SUMMARY OF THE INVENTION

The inventors have now surprisingly found the above problems may be solved by using a method for determining a percentage of recycled polyethylene terephthalate [r-PET, hereinafter] in a material [material M, hereinafter] with regards to the total amount of r-PET and non-recycled polyethylene terephthalate comprised in said material M, said r-PET being derived from at least one polymeric material comprising at least one PET polymer and optionally IPA monomers and optionally TPA monomers wherein said at least one PET polymer is derived from recurring units derived from ethylene glycol and recurring units derived from terephthalic acid [TPA, hereinafter] and recurring units derived from isophthalic acid [IPA, hereinafter]; said method comprising the steps of :

a) determining the molar ratio of IPA or the molar percentage of IPA of each reference material of a series of reference materials, each reference material comprising at least one PET polymer and optionally IPA monomers and optionally TPA monomers, wherein said at least one PET polymer is derived from recurring units derived from ethylene glycol and recurring units derived from TPA and recurring units derived from IPA; wherein the molar ratio of IPA of each reference material of said series of reference materials is the ratio of the molar quantity of the recurring units derived from IPA and when present the IPA monomers to the molar quantity of the recurring units derived from TPA and when present the TPA monomers as comprised in said each reference material of said series of reference materials; and wherein the molar percentage of IPA is the molar percentage of IPA recurring units and when present the IPA monomers as comprised in said each reference material of said series of reference materials;
b) calculating the average of the molar ratios of IPA or of the molar percentages of IPA of the series of reference materials as determined in step a);
c) providing a material M;
d) determining the molar ratio of IPA or the molar percentage of IPA of said material M by analysing said material M using at least one analysis method selected from the group consisting of nuclear magnetic resonance (NMR) spectroscopy, Fourier transform infrared (FTIR) spectroscopy, Raman spectroscopy, near infrared spectroscopy (NIR), gas chromatography (GC) and high pressure liquid chromatography (HPLC); wherein the molar ratio of IPA of said material M is the ratio of the molar quantity of the recurring units derived from IPA and when present the IPA monomers to the molar quantity of the recurring units derived from TPA and when present the TPA monomers as comprised in said material M; and wherein the molar percentage of IPA of said material M is the molar percentage of the IPA recurring units and when present the IPA monomers as comprised in said material M;
e) determining the percentage of r-PET in said material M by calculating the ratio $R_{IPA}$ wherein:

$$R_{IPA} = \frac{Y}{X} \star 100$$

and wherein X is the the average of the molar ratios of IPA or of the molar percentages of IPA of the series of reference materials as determined step b) and Y is the molar ratio of IPA or the molar percentage of IPA of said material M as determined in step d) and wherein X≥Y and X is different from 0.

The inventors have surprisingly found that such a method is an easy, rapid, versatile, and reproducible method for determining the percentage of recycled polyethylene terephthalate in a material with regards to the total amount of recycled PET and non-recycled PET comprised in said material.

DETAILED DESCRIPTION

Within the context of the present invention, the term "comprising" should not be interpreted as being restricted to the means listed thereafter; it does not exclude other elements or steps. It needs to be interpreted as specifying the presence of the stated features, integers, steps, or components as referred to, but does not preclude the presence or addition of one or more other features, integers, steps, or components, or groups thereof. Thus, the scope of the expression "a method comprising steps A and B" should not be limited to methods consisting only of steps A and B. It means that with respect to the present invention, the only relevant steps of the method are A and B. Accordingly, the terms "comprising" and "including" encompass the more restrictive terms "consisting essentially of" and "consisting of".

Within the context of the present invention, "PET" stands for polyethylene terephthalate.

Within the context of the present invention, the regioisomers IPA and TPA are intended to denote benzene-1,3-dicarboxylic acid and benzene-1,4-dicarboxylic acid, respectively.

Within the context of the present invention, r-PET material refers to PET material which is obtained by recycling PET containing materials according to any recycling processes. Non-limiting examples of said recycling processes notably include mechanical and chemical recycling processes and combinations of both. For example, said r-PET may be obtained from recycling or re-processing said at least one PET polymeric material.

Said r-PET is derived from at least one polymeric material comprising at least one PET polymer and optionally IPA monomers and optionally TPA monomers wherein said at least one PET polymer is derived from recurring units derived from ethylene glycol and recurring units derived from terephthalic acid [TPA, hereinafter] and recurring units derived from isophthalic acid [IPA, hereinafter].

Within the context of the present invention, TPA and IPA monomers refer respectively to free TPA and free IPA molecules. For example, the TPA and IPA monomers may be present in said at least one PET polymer as a result of the degradation of the at least one PET polymer. For example, the TPA and IPA monomers may also be TPA and IPA monomers that did not react during the synthesis of the at least one PET polymer. Depending on the analysis method and its resolution, it is not always possible to distinguish the recurring units derived from IPA from the IPA monomer and/or to distinguish the recurring units derived from TPA from the TPA monomer. As a consequence, in particular for some analysis method, a molar ratio of IPA is the ratio of the molar quantity of the recurring units derived from IPA and when present the IPA monomers to the molar quantity of the recurring units derived from TPA and when present the TPA monomers; and a molar percentage of IPA is the molar percentage of the IPA recurring units and when present the IPA monomers.

Said at least one polymeric material comprises at least one PET polymer and optionally TPA monomers and optionally IPA monomers. Said at least one PET polymer is derived from recurring units derived from ethylene glycol and recurring units derived from TPA and recurring units derived from IPA.

Said at least one PET polymeric material may be in any form known by the skilled in the art, for example in the form of packaging or containers such as bottles.

Generally, the units derived from IPA may be used to reduce the crystallinity of the polymers which is particularly advantageous when these polymers are used to make bottles via a stretch blow molding process because the obtained bottles are transparent but retain enough crystallinity to in order to prevent gases from passing through.

Preferably, said polymeric material comprises at least 75% by weight, more preferably at least 80% by weight, even more preferably 90 % by weight, even more preferably at least 95 % by weight, even more preferably at least 96 % by weight, even more preferably at least 97% by weight, even more preferably at least 98% by weight, even more preferably at least 99% by weight of said at least one PET polymeric material, with regards to the total weight of said polymeric material.

Said at least one PET polymer is derived from recurring units derived from ethylene glycol and recurring units derived from TPA and recurring units derived from IPA. Thus, the at least one PET polymer may be obtained by the copolymerization of ethylene glycol, TPA and IPA.

Preferably, said at least one PET polymer comprises at least 80% by weight, preferably at least 85% by weight, more preferably at least 90% by weight, even more preferably at least 95% by weight, by weight of recurring units derived from TPA and recurring units derived from ethylene glycol, with regards to the total weight of said at least one PET polymer.

Preferably, said at least one PET polymer comprises at most 99.5 % by weight, preferably at most 99 % by weight, more preferably at most 98 % by weight, even more preferably at most 97% by weight of recurring units derived from TPA and recurring units derived from ethylene glycol, with regards to the total weight of said at least one PET polymer.

In a preferred embodiment, said at least one PET polymer comprises at least 80% by weight and at most 99.5% by weight, preferably at least 85% by weight and at most 99% by weight, more preferably at least 90% by weight and at most 98% by weight, even more preferably at least 95% by weight and at most 97% by weight of recurring units derived from TPA and recurring units derived from ethylene glycol, with regards to the total weight of said at least one PET polymer.

Preferably, said at least one PET polymer comprises at least 0.5 % by weight, preferably at least 0.8 % by weight, more preferably at least 1 % by weight, even more preferably at least 1.5 % by weight, by weight of recurring units derived from IPA, with regards to the total weight of said at least one PET polymer.

Preferably, said at least one PET polymer comprises at most 15 % by weight, preferably at most 10% by weight, more preferably at most 8 % by weight, even more preferably at most 5 % by weight of recurring units derived from IPA, with regards to the total weight of said at least one PET polymer.

In a preferred embodiment, said at least one PET polymer comprises at least 0.5 % by weight and at most 15 % by weight, preferably at least 0.8 % by weight and at most 10 % by weight, more preferably at least 1 % by weight and at most 5 % by weight, even more preferably at least 1.5 % by weight and at most 5 % by weight of recurring units derived from IPA, with regards to the total weight of said at least one PET polymer.

In a preferred embodiment, said r-PET is obtained by recycling said at least one polymeric material, said at least one polymeric material being in the form of bottles comprising at least one PET polymer comprising, relative to the total weight of said at least one PET polymer, at least 80% by weight and at most 99.5% by weight of recurring units derived from ethylene glycol and recurring units derived from TPA and at least 0.5 % and at most 15 % by weight recurring units derived from IPA.

In step a), the molar ratio of IPA or the molar percentage of IPA of each reference material of a series of reference materials is determined. Each reference material comprises at least one PET polymer and optionally IPA monomers and optionally TPA monomers, wherein said at least one PET polymer is derived from recurring units derived from ethylene glycol and recurring units derived from TPA and recurring units derived from IPA. The molar ratio of IPA of each reference material of said series of reference materials is the ratio of the molar quantity of the recurring units derived from IPA and when present the IPA monomers to the molar quantity of recurring units derived from TPA and when present TPA monomers as comprised in said each reference material of said series of reference materials. The molar percentage of IPA is the molar percentage of IPA recurring units and when present the IPA monomers as comprised in said each reference material of said series of reference materials.

The step a) of determining the molar ratio of IPA or the molar percentage of IPA of each reference material of a series of reference materials, can be achieved by using any techniques known by the skilled in the art. Non limiting examples of such techniques include NMR spectroscopy, FTIR spectroscopy, Raman spectroscopy, GC, NIR and HPLC.

Preferably, said molar ratio of IPA or said molar percentage of IPA of each reference material of a series of reference materials is determined by using $^1$H-NMR. More preferably, said molar ratio of IPA or said molar percentage of IPA of each reference material of a series of reference materials is determined by using $^1$H-NMR and a deuterated solvent selected from the group consisting of deuterated trifluoroacetic acid (TFA-d$_1$), deuterated 1,1,2,2-tetrachloroethane-d$_2$, deuterated dimethyl sulfoxide-d$_6$ (DMSO-d$_6$) and mixtures thereof. Even more preferably, said molar ratio of IPA or said molar percentage of IPA of each reference material of a series of reference materials is determined by using $^1$H-NMR and deuterated TFA.

Within the context of the present invention, $^1$H-NMR encompasses liquid state $^1$H-NMR and solid state $^1$H-NMR.

The composition of said reference materials may for example change according to their production process, the country or region and/or the production plant where they are made, due for example to local regulations or other circumstances.

Thus, preferably, said series of reference materials is from the same the country or region and/or the production plant as said material M. It has been observed that by doing so, the method according to the present invention displays improved accuracy.

The molar percentage of IPA of each reference material of a series of reference materials may make reference to different kind of molar percentages.

For example, the molar percentage of IPA may be the molar percentage of the recurring units derived from IPA and when present the IPA monomers with respect to the total amount of recurring units and when present monomers as comprised in each reference material of a series of reference materials. In that case, the total amount of recurring units includes the recurring units derived from IPA, the recurring units derived from TPA and the recurring units derived from ethylene glycol and the total amount of monomers include IPA monomers, TPA monomers and ethylene glycol monomers.

Alternatively, the molar percentage of IPA may be the molar percentage of the recurring units derived from IPA and when present IPA monomers with respect to the total amount of the recurring units derived from IPA and the recurring units derived from TPA and when present the IPA monomers and when present the TPA monomers as comprised in each reference material of a series of reference materials.

Each reference material comprises at least one PET polymer and optionally IPA monomers and optionally TPA monomers; said at least one PET polymer is derived from the recurring units derived from ethylene glycol and the recurring units derived from TPA and the recurring units derived from IPA.

Preferably, said each reference material comprises at least 90 % by weight, more preferably at least 95 % by weight, even more preferably at least 96 % by weight, even more preferably at least 97% by weight, even more preferably at least 98% by weight, even more preferably at least 99% by weight of said at least one PET polymer, with regards to the total weight of said each reference material.

Preferably, said at least one PET polymer comprised in said each reference material, comprises at least 80% by weight, preferably at least 85% by weight, more preferably at least 90% by weight, even more preferably at least 95% by weight, by weight of the recurring units derived from TPA and the recurring units derived from ethylene glycol, with regards to the total weight of said at least one PET polymer.

Preferably, said at least one PET polymer comprised in said each reference material, comprises at most 99.5 % by weight, preferably at most 99 % by weight, more preferably at most 98 % by weight, even more preferably at most 97% by weight of the recurring units derived from TPA and the recurring units derived from ethylene glycol, with regards to the total weight of said at least one PET polymer.

In a preferred embodiment, said at least one PET polymer comprised in said each reference material, comprises at least 80% by weight and at most 99.5% by weight, preferably at least 85% by weight and at most 99% by weight, more preferably at least 90% by weight and at most 98% by weight, even more preferably at least 95% by weight and at most 97% by weight of the recurring units derived from TPA and the recurring units derived from ethylene glycol, with regards to the total weight of said at least one PET polymer.

Preferably, said at least one PET polymer comprised in said each reference material comprises at least 0.5 % by weight, preferably at least 0.8 % by weight, more preferably at least 1 % by weight, even more preferably at least 1.5 % by weight, by weight of the recurring units derived from IPA, with regards to the total weight of said at least one PET polymer.

Preferably, said at least one PET polymer comprised in said each reference material comprises at most 15 % by weight, preferably at most 10 % by weight, more preferably at most 8 % by weight, even more preferably at most 5 % by weight of the recurring units derived from IPA, with regards to the total weight of said at least one PET polymer.

In a preferred embodiment, said at least one PET polymer comprised in said each reference material, comprises at least 0.5 % by weight and at most 15 % by weight, preferably at least 0.8 % by weight and at most 10 % by weight, more preferably at least 1 % by weight and at most 5 % by weight, even more preferably at least 1.5 % by weight and at most 5 % by weight of the recurring units derived from IPA, with regards to the total weight of said at least one PET polymer.

In step c), the average of the molar ratios of IPA or the average of the molar percentage of IPA of the series of reference materials as determined in step a) is an arithmetic average.

If desired, the molar ratio of IPA or the molar percentage of IPA of each reference material of the series of reference materials and/or the average X may be stored in a storage device in order to build a reference library of molar ratios of IPA. Said reference library may preferably be stored in a data storage device, such as a hard drive. Preferably, the reference library may also contain the precise region of origin of the series of reference material, the time at which the reference materials were produced and/or preferably any other information deemed necessary by the skilled in the art.

In step c), a material M is provided.

Any materials may be analysed by the method according to the present invention. Non-limiting examples of said materials comprise pieces of fabrics, toys, or any consumer grade objects.

In particular, said material M comprise at least one PET polymer comprising recurring units derived from ethylene glycol and recurring units derived from TPA. Said material M may also be in the form of fibers, more particularly woven or non-woven fibers.

If desired, said material M may further comprise additional materials such as cotton, viscose or preferably any suitable material.

If desired, said material M may comprise at least 5% by weight, preferably at least 10 % by weight, more preferably at least 20 % by weight, even more preferably at least 30% by weight, even more preferably at least 40% by weight, even more preferably at least 50 % by weight, even more preferably at least 60% by weight, even more preferably at least 70% by weight, even more preferably at least 80% by weight, even more preferably at least 85 % by weight, even more preferably at least 90 % by weight, even more preferably at least 95 % by weight, even more preferably at least 96 % by weight, even more preferably at least 97% by weight, even more preferably at least 98% by weight, even more preferably at least 99% by weight of said at least one PET polymer, with regards to the total weight of said material M.

Preferably, said material M is at least partially or totally soluble in organic solvent, more preferably at least partially or totally soluble in a solvent selected from the group consisting of tetrachloroethane, DMSO, TFA, TCA and mixtures thereof.

The molar ratio of IPA or the molar percentage of IPA of said material M by analysing said material M is determined using at least one analysis method selected from the group consisting of nuclear magnetic resonance (NMR) spectroscopy, Fourier transform infrared (FTIR) spectroscopy, Raman spectroscopy, gas chromatography (GC), high pressure liquid chromatography (HPLC) and near infrared spectroscopy (NIR); wherein the molar ratio of IPA of said material M is the ratio of the molar quantity of the recurring units derived from IPA and when present the IPA monomers to the molar quantity of the recurring units derived from TPA and when present the TPA monomers as comprised in said material M; and wherein the molar percentage of IPA of said material M is the molar percentage of IPA recurring units and when present the IPA monomers as comprised in said material M.

The molar percentage of IPA of said material M may make reference to different kind of molar percentages.

For example, the molar percentage of IPA of said material M may be the molar percentage of the recurring units derived from IPA and when present the IPA monomers with respect to the total amount of recurring units and when present monomers as comprised in said material M. In that case, the total amount of recurring units includes recurring units derived from IPA, recurring units derived from TPA and recurring units derived from ethylene glycol and the total amount of monomers include the IPA monomers, the TPA monomers and the ethylene glycol monomers.

Alternatively, the molar percentage of IPA of said material M may be the molar percentage of the recurring units derived from IPA and when present the IPA monomers with respect to the total amount of the recurring units derived from IPA and the recurring units derived from TPA and when present the TPA monomers as comprised in said material M.

X is the average of the molar ratios of IPA or of the molar percentages of IPA of the series of reference materials as determined step b) and Y is the molar ratio of IPA or the molar percentage of IPA of said material M as determined in step d).

Hereafter, the molar ratio of IPA in said material M may be referred to the molar ratio Y and the molar percentage of IPA in said material M may be referred to as the molar percentage Y.

Preferably, said molar ratio Y or said molar percentage Y is determined by analysing said material M by using NMR, more preferably $^1$H-NMR, even more preferably liquid state $^1$H-NMR.

Preferably, said molar ratios of IPA of each of the reference material of said series of reference materials is determined by analysing said each of the reference material using NMR, more preferably $^1$H-NMR, even more preferably liquid state $^1$H-NMR.

Preferably, before the step of determining said molar ratio Y or said molar percentage Y and/or the step of determining the molar ratios of IPA of each of the reference material of said series of reference materials, said material M and/or said each reference material is at least partially or totally dissolved in a deuterated solvent selected from the group consisting of deuterated trifluoroacetic acid (TFA-$d_1$), deuterated 1,1,2,2-tetrachloroethane-$d_2$, deuterated dimethyl sulfoxide-$d_6$ (DMSO-$d_6$) and mixtures thereof.

More preferably, said material M is at least partially or totally dissolved in a deuterated solvent, thereby creating a mixture comprising at least 20 % by weight, preferably at least 30% by weight, more preferably at least 40 % by weight of said material M, relative to the total weight of said mixture. More preferably, said material M is at least partially or totally dissolved in a deuterated solvent, thereby creating a mixture comprising at most 80 % by weight, preferably at most 70% by weight, more preferably at most 60 % by weight of said material, relative to the total weight of said mixture. Preferably, said deuterated solvent may be selected from the group consisting of deuterated trifluoroacetic acid (TFA-$d_1$), deuterated 1,1,2,2-tetrachloroethane-$d_2$, deuterated dimethyl sulfoxide-$d_6$ (DMSO-$d_6$) and mixtures thereof.

In a preferred embodiment, said material M and/or said each reference material is at least partially or totally dissolved in a deuterated solvent, thereby creating a mixture comprising at least 20 % by weight and at most 80 % by weight, preferably at least 30% by weight and at most 70% by weight, more preferably at least 40 % by weight and at most 60 % by weight of said material M and/or said each reference material, relative to the total weight of said mixture. Preferably, said deuterated solvent may be selected from the group consisting of deuterated trifluoroacetic acid (TFA-$d_1$), deuterated 1,1,2,2-tetrachloroethane-$d_2$, deuterated dimethyl sulfoxide-$d_6$ (DMSO-$d_6$) and mixtures thereof.

In particular, said each reference material is at least partially or totally dissolved in a deuterated solvent, thereby creating a mixture comprising at least 20 % by weight, preferably at least 30% by weight, more preferably at least 40 % by weight of each reference material, relative to the total weight of said mixture. In particular, said each reference material is at least partially or totally dissolved in a deuterated solvent, thereby creating a mixture comprising at most 80 % by weight, preferably at most 70% by weight, more preferably at most 60 % by weight of said each reference material, relative to the total weight of said mixture. Preferably, said deuterated solvent may be selected from the group consisting of deuterated trifluoroacetic acid (TFA-$d_1$), deuterated 1,1,2,2-tetrachloroethane-$d_2$, deuterated dimethyl sulfoxide-$d_6$ (DMSO-$d_6$) and mixtures thereof.

In a preferred embodiment, said each reference material is at least partially or totally dissolved in a deuterated solvent, thereby creating a mixture comprising at least 20 % by weight and at most 80 % by weight, preferably at least 30% by weight and at most 70% by weight, more preferably at least 40 % by weight and at most 60 % by weight of said each reference material, relative to the total weight of said mixture. Preferably, said deuterated solvent may be selected from the group consisting of deuterated trifluoroacetic acid (TFA-$d_1$), deuterated 1,1,2,2-tetrachloroethane-$d_2$, deuterated dimethyl sulfoxide-$d_6$ (DMSO-$d_6$) and mixtures thereof.

If desired, after being dissolved at least partially or totally in said deuterated solvent, said dissolved material M and/or the dissolved reference material may be filtered.

Preferably, said molar ratios of IPA of each of the reference material of said series of reference materials and/or said molar ratio Y or said molar percentage Y is determined using NMR spectroscopy, more preferably $^1$H-NMR spectroscopy, even more preferably liquid state $^1$H-NMR.

Preferably, $^1$H-NMR spectroscopy is used with a zgig pulse program. A zgig pulse program is known by the skilled in the art and is a 90° pulse with a 1D-sequence with inverse gated decoupling. Other suitable pulse program may be used if they are suitable for determining the molar ratios of IPA of each of the reference material of said series of reference materials and/or said molar ratio Y.

Preferably, for determining the molar ratios of IPA of each of the reference material of said series of reference materials and/or said molar ratio Y or said molar percentage Y, $^1$H-NMR spectroscopy is used with a D1 delay of at least 5 s or at least 10 s, or at least 15 s, or at least 20 s. Preferably, $^1$H-NMR is used with a D1 delay of at most 40 s or at most 30 s, or at most 20 s, or at most 15 s.

In a preferred embodiment, for determining the molar ratios of IPA of each of the reference material of said series of reference materials and/or said molar ratio Y or said molar percentage Y, $^1$H-NMR spectroscopy is used with a D1 delay of at least 5 s and at most 40 s, preferably at least 5 s and at most 30 s, more preferably at least 5 s and at most 20 s, more preferably at least 10 s and at most 20 s.

The D1 delay is known by the skilled in the art and is the relaxation delay.

Preferably, for determining the molar ratios of IPA of each of the reference material of said series of reference materials and/or said molar ratio Y or said molar percentage Y, $^1$H-NMR spectroscopy is used with a time domain (TD) of at least 20k, more preferably at least 30k, even more preferably at least 32k points. Preferably, $^1$H-NMR spectroscopy is used with a time domain (TD) of at most 45k, more preferably at most 40k, even more preferably at most 35k points.

In a preferred embodiment, for determining the molar ratios of IPA of each of the reference material of said series of reference materials and/or said molar ratio Y or said molar percentage Y, $^1$H-NMR spectroscopy is used with a time domain (TD) of at least 20k and at most 45k, more preferably at least 30k and at most 40k, even more preferably at least 32k and at most 35k points.

Preferably, for determining the molar ratios of IPA of each of the reference material of said series of reference materials and/or said molar ratio Y or said molar percentage Y, $^1$H-NMR spectroscopy is used with a sweep width (SW) of at least 10 ppm, more preferably at least 12 ppm, more preferably at least 15 ppm. Preferably, $^1$H-NMR spectroscopy is used with a sweep width (SW) of at most 20 ppm, more preferably at most 17 ppm, more preferably at most 16 ppm.

In a preferred embodiment, for determining the molar ratios of IPA of each of the reference material of said series of reference materials and/or said molar ratio Y or said molar percentage Y, $^1$H-NMR spectroscopy is used with a sweep width (SW) of at least 10 ppm and at most 20 ppm, more preferably at least 12 ppm and at most 17 ppm, more preferably at least 15 ppm and at most 16 ppm.

It is understood that the Sweep width (SW) and time domain (TD) can be adjusted if needed so that the resulting spectral window contains the -0.5 to 10 ppm region.

Preferably, for determining the molar ratios of IPA of each of the reference material of said series of reference materials and/or said molar ratio Y or said molar percentage Y, $^1$H-NMR spectroscopy is used with a number of transients (NS) of at least 10, more preferably at least 20, even more preferably at least 30, even more preferably at least 32. Preferably, $^1$H-NMR spectroscopy is used with a number of transients (NS) of at most 50, more preferably at most 40, even more preferably at most 35.

In a preferred embodiment, for determining the molar ratios of IPA of each of the reference material of said series of reference materials and/or said molar ratio Y or said molar percentage Y, $^1$H-NMR spectroscopy is used with a number of transients (NS) of at least 10 and at most 50, more preferably at least 20 and at most 40, even more preferably at least 30 and at most 35, even more preferably at least 32 and at most 35.

Preferably the step of determining said the molar ratios of IPA of each of the reference material of said series of reference materials by analysing said each reference material of said series of reference materials and/ or the step of determining said molar ratio Y by analysing said material M comprises a step of:

- acquiring at least one $^1$H-NMR spectrum of said each reference material of said series of reference materials or said material M,
- calculating in said at least one $^1$H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA,
- calculating in said at least one $^1$H-NMR spectrum, the integral of the triplet peak [hereinafter, A2] corresponding to one aromatic proton of the recurring units derived from IPA, in particular, the triplet peak corresponds to the aromatic proton at the position 5 of the aromatic ring of said recurring units derived from IPA,
- determining said molar ratio Y or the molar ratios of IPA of each of the reference material by applying formula (II):

$$\frac{4 * A2}{(A1 - (2 * A2))}$$

formula (II)

Alternatively, the step of determining said the molar ratios of IPA of each of the reference material of said series of reference materials by analysing said each reference material of said series of reference materials and/ or the step of determining said molar ratio Y by analysing said material M comprises a step of:

- acquiring at least one $^1$H-NMR spectrum of said each reference material of said series of reference materials or said material M,
- calculating in said at least one $^1$H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA;
- calculating in said at least one $^1$H-NMR spectrum, the integral of the singlet peak [hereinafter, A3] corresponding to one aromatic proton of the recurring units derived from IPA, in particular the singlet peak corresponds to the aromatic proton at the position 2 of the aromatic ring of said recurring units derived from IPA,
- determining said molar ratio Y or the molar ratios of IPA of each of the reference material by applying formula (III):

$$\frac{4 * A3}{(A1 - (2 * A3))}$$

formula (III)

In another alternative embodiment, the step of determining said the molar ratios of IPA of each of the reference material of said series of reference materials by analysing said each reference material of said series of reference materials and/ or the step of determining said molar ratio Y by analysing said material M comprises a step of:

- acquiring at least one $^1$H-NMR spectrum of said each reference material of said series of reference materials or said material M,
- calculating in said at least one $^1$H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA,
- calculating in said at least one $^1$H-NMR spectrum, the integral of the singlet peak [hereinafter, A3] corresponding to one aromatic proton of the recurring units derived from IPA, in particular the singlet peak corresponds to the aromatic proton at the position 2 of the aromatic ring of said recurring units derived from IPA,
- calculating in said at least one $^1$H-NMR spectrum, the integral of the triplet peak [hereinafter, A2] corresponding to one aromatic proton of the recurring units derived from IPA, in particular the triplet peak corresponds to the aromatic proton at the position 5 of the aromatic ring of said recurring units derived from IPA,
- determining said molar ratio Y or the molar ratios of IPA of each of the reference material by applying formula (IV) or formula (V):

$$\frac{4 * A3}{(A1 - (2 * A2))}$$

Formula (IV)

$$\frac{4 * A2}{(A1 - (2 * A3))}$$

Formula (V)

In the context of the present invention, the terms "calculating the integral" mean to determine the relative area under the curve. The skilled in the art knows how to calculate an integral.

For example, calculating the integral A1 of the peak corresponding to the four aromatic protons of the recurring units derived from TPA and the doublet peak corresponding to two aromatic protons of the recurring units derived from IPA, can be achieved by calculating an integral of a single region of the spectrum comprising the peak corresponding to the four aromatic protons of the recurring units derived from TPA and the doublet peak corresponding to two aromatic protons of the recurring units derived from IPA.

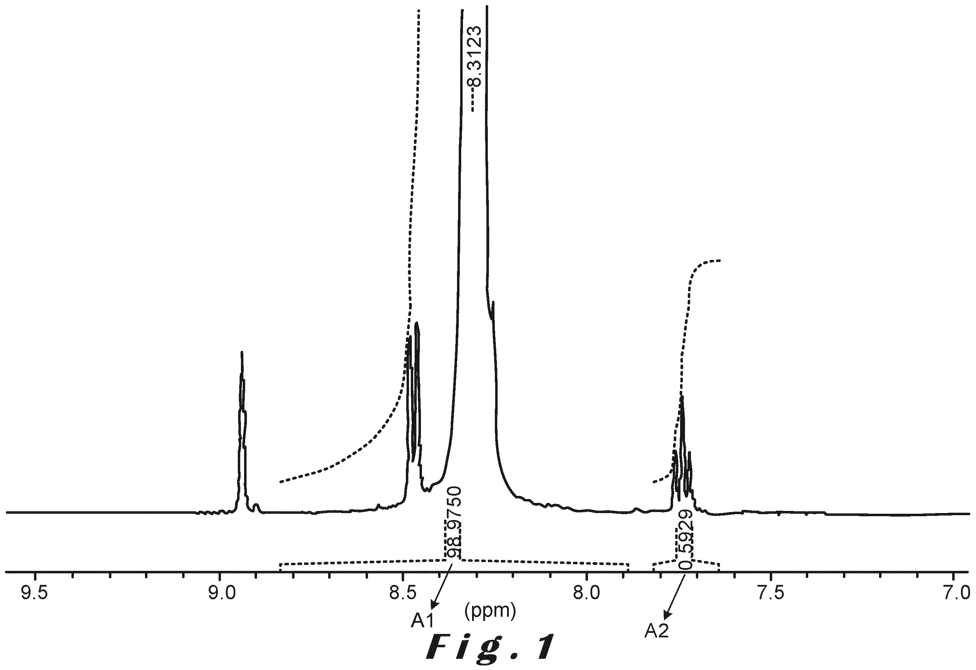

Figure 1 illustrates a $^1$H-NMR spectrum in deuterated TFA in the region 7-9.5 ppm and the A1 and A2 integrals.

Preferably, said molar ratio Y or said molar percentage Y and/ or the molar ratios of IPA of each of the reference material of said series of reference materials can be determined by analysing said material M using any NMR spectrometer, preferably a 300 MHz NMR spectrometer, or a 400 MHz NMR spectrometer or a 500 MHz NMR spectrometer.

The percentage of r-PET in said material M is determined by calculating the ratio $R_{IPA}$ as defined in formula (I):

$$R_{IPA} = \frac{Y}{X} * 100$$

(Formula I)

wherein X is the the average of the molar ratios of IPA or of the molar percentages of IPA of the series of reference materials as determined step b) and Y is the molar ratio of IPA or the molar percentage of IPA of said material M as determined in step d) and wherein wherein X≥Y and X is different from 0. $R_{IPA}$ is expressed in %.

When X is the the average of the molar ratios of IPA of the series of reference materials, Y is the molar ratio of IPA of said material M.

When X is the average of the molar percentages of IPA of the series of reference materials, Y is the molar percentage of IPA of said material M.

Generally speaking, with regards to said material M or said series of reference materials, the molar percentage of IPA may be the molar percentage of the recurring units derived from IPA and when present the IPA monomers with respect to the total amount of recurring units and when present the monomers. In that case, the total amount of recurring units include the recurring units derived from IPA, the recurring units derived from TPA and the recurring units derived from ethylene glycol and the total amount of monomers include the IPA monomers, the TPA monomers and the ethylene glycol monomers.

Alternatively, the molar percentage of IPA may be the molar percentage of the recurring units derived from IPA and when present IPA monomers with respect to the total amount of the recurring units derived from IPA and the recurring units derived from TPA and when present the IPA monomers and when present the TPA monomers.

When the content of IPA is very small in comparison to the content of the TPA, using the present method with the molar percentage of IPA or the molar ratio of IPA will yield almost the same result within the appropriate limit of measurement accuracy.

Other equivalents include, using the present method with the inverse of the molar percentage of IPA or the inverse of the molar ratio of IPA, in which cases the $R_{IPA}$ must also be inversed to yield the correct result.

When analyzing a material M by $^1$H-NMR, there is less chance to observe additional signals in the aromatic region of the spectrum attributed to other materials or impurities. However, it is likely that other peaks attributed to other materials or impurities appear in the region of 3 to 4 ppm where the signals of the recurring units derived from ethylene glycol can be observed.

Thus, the method according to the present invention has improved accuracy and reliability when the method is based on the molar ratio of IPA as defined above or when the method is based on the molar percentage of IPA calculated as the molar percentage of the recurring units derived from IPA and when present the IPA monomers with respect to the total amount of recurring units derived from IPA and the recurring units derived from TPA and when present IPA monomers and when present the TPA monomers.

The molar ratios or molar percentages of IPA of each of said reference material and the molar ratio Y or said molar percentage Y are preferably determined by using the same analysis method. The inventors have surprisingly found that it improves the accuracy of the method according to the present invention.

Preferably the step of determining said the molar percentages of IPA of each of the reference material of said series of reference materials by analysing said each reference material of said series of reference materials and/ or the step of determining said molar percentage Y by analysing said material M comprises a step of:

- acquiring at least one $^1$H-NMR spectrum of said each reference material of said series of reference materials or said material M,
- calculating in said at least one $^1$H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA,
- calculating in said at least one $^1$H-NMR spectrum, the integral of the triplet peak [hereinafter, A2] corresponding to one aromatic proton of the recurring units derived from IPA, in particular, the triplet peak corresponds to the aromatic proton at the position 5 of the aromatic ring of said recurring units derived from IPA,
- determining said molar percentage Y or the molar percentages of IPA of each of the reference material by applying formula (VI):

$$\frac{4 * A2}{(A1 - (2 * A2) + (4 * A2))}$$

formula (VI)

Alternatively, the step of determining said the molar percentages of IPA of each of the reference material of said series of reference materials by analysing said each reference material of said series of reference materials and/ or the step of determining said molar percentage Y by analysing said material M comprises a step of:

- acquiring at least one $^1$H-NMR spectrum of said each reference material of said series of reference materials or said material M,

- calculating in said at least one $^1$H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA;

- calculating in said at least one $^1$H-NMR spectrum, the integral of the singlet peak [hereinafter, A3] corresponding to one aromatic proton of the recurring units derived from IPA, in particular the singlet peak corresponds to the aromatic proton at the position 2 of the aromatic ring of said recurring units derived from IPA,
- determining said molar percentage Y or the molar percentages of IPA of each of the reference material by applying formula (VII):

$$\frac{4 * A3}{(A1 - (2 * A3) + (4 * A3))}$$

formula (VII)

In another alternative embodiment, the step of determining said the molar percentages of IPA of each of the reference material of said series of reference materials by analysing said each reference material of said series of reference materials and/ or the step of determining said molar percentage Y by analysing said material M comprises a step of:

- acquiring at least one $^1$H-NMR spectrum of said each reference material of said series of reference materials or said material M,
- calculating in said at least one $^1$H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA,
- calculating in said at least one $^1$H-NMR spectrum, the integral of the singlet peak [hereinafter, A3] corresponding to one aromatic proton of the recurring units derived from IPA, in particular the singlet peak corresponds to the aromatic proton at the position 2 of the aromatic ring of said recurring units derived from IPA,
- calculating in said at least one $^1$H-NMR spectrum, the integral of the triplet peak [hereinafter, A2] corresponding to one aromatic proton of the recurring units derived from IPA, in particular the triplet peak corresponds to the aromatic proton at the position 5 of the aromatic ring of said recurring units derived from IPA,
- determining said molar percentage Y or the molar percentages of IPA of each of the reference material by applying formula (VIII) or formula (IX):

$$\frac{4 * A3}{(A1 - (2 * A2) + (4 * A3))}$$

Formula (VIII)

$$\frac{4 * A2}{(A1 - (2 * A3) + (4 * A2))}$$

Formula (IX)

**A method for determining a percentage of recycled polyethylene terephthalate by Fourier transform infrared (FTIR) spectroscopy**

General procedure of sample preparation for FTIR measurements

Firstly, the PET sample can be ground into powder then mixed with an amount of KBr power. The obtained mixture can be then compressed into a pellet. The process for making KBr pellets for FTIR measurements is common general knowledge for the skilled in the art.

## General procedure for FTIR measurements

The FTIR spectroscopy measurement can be carried out using a Nicolet iS10 Transmission Spectrometer or equivalent.

A background can be first acquired after setting the background scan number to 64.

For each analyzed PET sample (prepared into KBr pellet), a FTIR spectrum in absorption mode can be acquired. The number of scans can be set to 64.

## FTIR Calibration

The calibration can be carried out using standards which are PET samples with known amounts of IPA, preferably with 0.9 mol%, 2.3 mol%, 4.7 mol% and 9.4 mol% of IPA.

For each standard, KBr pellets can be prepared according to the General procedure described above.

For each standard prepared in KBr pellet, the FTIR spectrum in absorption mode can be acquired according to the general procedure for FTIR measurement above. For all the standard, the normalization can be carried out considering the whole spectra or peaks area at 1300, 1450, 1630 and 3100 cm$^{-1}$.

The Agilent's software builds a calibration by applying a linear regression method such as a multiple linear least squares regression (MLR). Agilent's software is "MicroLab Quant for quantitative method development" revision 5.6.2064 of 2019.

## Determination of the IPA molar percentage Y of a material M

A material M which is a PET fibre for use in a textile with an unknown IPA molar percentage can be provided.

The FTIR spectrum of said material M can be recorded in the same conditions using the same conditions, method and apparatus than for the FTIR measurements of the PET standards for the above FTIR calibration.

The Agilent's software can determine the molar percentages of IPA Y in said material M by using the MLR calibration which was obtained above.

## Determination of the average X of the molar percentages of IPA of the series of reference materials

A series of reference material which contains recycled PET flakes originating from one commercial type of PET beverage bottles belonging to a well-known trademark can be provided.

For each reference material, the molar percentages of IPA can be determined by FTIR using the same determination method which was used above for the determination of the IPA molar percentages of the material M (i.e. via a MLR calibration using MicroLab Quant for quantitative method development of Agilent revision 5.6.2064 of 2019).

Then the average X of the IPA molar percentages of said series of reference materials can be calculated.

## Determination of the percentage of r-PET in said material M

The percentage of r-PET in said material M is calculated by using the ratio $R_{IPA}$ wherein:

$$R_{IPA} = \frac{Y}{X} * 100$$

and wherein X is the average of the molar percentages of IPA of the series of reference materials and Y is the molar percentage of IPA of said material M.

## A method for determining a percentage of recycled polyethylene terephthalate by Raman spectroscopy

### General procedure for Raman measurements

The Raman spectroscopy measurement can be carried out using the SmartRaman DXR3 from Thermo Scientific or equivalent.

A background can be first acquired after setting the background scan number to 64. For each analyzed PET sample, a Raman spectrum in absorption mode can be acquired. The number of scans can be set to 64.

Raman Calibration

The calibration can be carried out using standards which are PET samples with known amounts of IPA, preferably with 0.9 mol%, 2.3 mol%, 4.7 mol% and 9.4 mol% of IPA.

For each PET sample, the Raman spectrum can be acquired according to the general procedure for Raman measurement above.

In each Raman spectrum, the peak at around 1125 cm$^{-1}$ corresponds to the benzene vibration modes of TPA, while the peak at around 1002 cm$^{-1}$ corresponds to the benzene vibration modes of IPA.

The calibration curve can be obtained by plotting the ratio $A_{1002}/A_{1125}$ as a function of the IPA mole percentage for each material Ma of a serie of materials Ma, wherein $A_{1002}$ and $A_{1125}$ are respectively the Raman intensity of the peaks corresponds to the benzene vibration modes of IPA and TPA. The calibration curve can be obtained by applying a regression method based on the least square method, known by the skilled in the art.

Determination of the IPA molar percentage Y of a material M

A material M which can be a PET fibre for use in a textile with an unknown IPA molar percentage can be provided.

The Raman spectrum of said material M can be recorded in the same conditions using the same conditions, method and apparatus than for the Raman measurements of the PET standards for the above Raman calibration.

The IPA molar percentage of Y of the material M can be determined by Raman spectroscopy by the following formula:

$$Y = \alpha \frac{A_{1002}}{A_{1002} + \beta A_{1125}}$$

<div align="right">Formula X</div>

wherein $A_{1002}$ and $A_{1125}$ are respectively the Raman intensity of the peaks corresponds to the benzene vibration modes of IPA and TPA. $\alpha$ and $\beta$ are coefficients which are derived from the calibration curve. The calibration curve can be considered linear for low IPA molar percentages (less than 10 mol.%).

Determination of the average X of the molar percentages of IPA of the series of reference materials

A series of reference material which contains recycled PET flakes originating from one commercial type of PET beverage bottles belonging to a well-known trademark is provided.

For each reference material, the molar percentages of IPA can be determined by Raman using the same determination method which was used above for the determination of the IPA molar percentages of the material M.

Then the average X of the IPA molar percentages of said series of reference materials can be calculated.

Determination of the percentage of r-PET in said material M

The percentage of r-PET in said material M is calculated by using the ratio $R_{IPA}$ wherein:

$$R_{IPA} = \frac{Y}{X} \star 100$$

and wherein X is the average of the molar percentages of IPA of the series of reference materials and Y is the molar percentage of IPA of said material M.

**A method for determining a percentage of recycled polyethylene terephthalate by gas chromatography (GC)**

General procedure for samples preparation for GC analysis

In order to analyse PET samples by GC, it is necessary to breakdown the PET polymer chains into more volatile smaller molecules. This can be achieved by methanolysis. During methanolysis, all the IPA recurring units and TPA recurring units are transformed into respectively dimethylisophthalate and dimethylterephthalate. As a consequence, measuring the molar percentages of dimethylisophthalate is equivalent to measuring the molar percentages of isophthalic acid.

A PET sample (for example 0.3 g) such as a piece of PET bottle or PET fibre can be mixed with NaOH in 5 ml of methanol in a closed reactor. The NaOH can be present in 1 % by weight based on the total weight of the NaOH and the methanol. The PET samples can be obtained through gridding until the PET particles reached sizes between 350 and 430 $\mu$m. In this particular case, the size of the PET particles refers to the greatest dimension of the particle. The mixture can then be heated inside the closed reactor at a temperature of 150°C for at least 30 minutes but not more than 45 minutes. The obtained clear solution can then be analysed by GC.

General conditions for GC measurements

The GC measurements can be carried out on a Hewlett Packard model HP 6890 or equivalent, performed on DB-Wax column model J&W125-7062, column length 60.0 m diameter 530 $\mu$m, 1.00 $\mu$m film thickness with flame-ionisation detector.

Conditions at inlet: 250 °C temperature, 20.00 psi pressure, 50:1 split ratio, 583.4 ml/min split flow with He as carrier gas.
Temperature program: initial temperature 170°C, 20°C/min until 235°C holding time 10 min then decrease 20°C/min until 170°C.
Conditions at detector: 250°C.
Hydrogen flow: 30.0 ml/min, airflow: 300 ml/min, Makeup Gas type: nitrogen, makeup flow: 23.0 ml/min.
Chromatogram recording time: at least 25min

A blank can also be used:5 ml of the NaOH mixture in methanol, which undergoes the same treatment as the sample. This makes possible to demonstrate the absence of interferences due to the treatment before analyses.

GC Calibration

The calibration can be carried out using standards which are PET samples with known amounts of dimethylisophthalate, more preferably with 0.9 mol%, 2.3 mol%, 4.7 mol% and 9.4 mol% of dimethylisophthalate. Diethylphthalate is used as internal standard.
The standards can be analysed by GC and a calibration curve can be constructed which correlates the peak area ratio with the IPA mole percentage concentration.

Determination of the IPA molar percentage Y of a material M by GC

A material M which is a PET fibre for use in a textile with an unknown IPA molar percentage is provided.
The material M can be prepared according to the General procedure for samples preparation as defined above.
The mixture obtained after applying the general procedure for samples preparation to the material M can be analysed by GC using the general conditions for GC analysis as defined above with diethylphthalate as internal standard. The GC chromatograph of the mixture resulting from the treatment of material M can be recorded.
Then, the IPA molar percentage Y of said material M can be obtained via the GC calibration above.

Determination of the average X of the molar percentages of IPA of the series of reference materials by GC

A series of reference material which contains recycled PET flakes originating from one commercial type of PET beverage bottles belonging to a well-known trademark can be provided.
For each reference material, the molar percentages of IPA can be determined by GC using the same determination method which was used above for the determination of the IPA molar percentages of the material M by GC.
Then the average X of the IPA molar percentages of said series of reference materials can be calculated.

Determination of the percentage of r-PET in said material M

The percentage of r-PET in said material M is calculated by using the ratio $R_{IPA}$ wherein:

$$R_{IPA} = \frac{Y}{X} * 100$$

and wherein X is the average of the molar percentages of IPA of the series of reference materials as determined by GC and Y is the molar percentage of IPA of said material M as determined by GC.

**A method for determining a percentage of recycled polyethylene terephthalate by High Pressure Liquid Chromatography (HPLC)**

General procedure for samples preparation

In order to analyse PET samples by HPLC, it is necessary to breakdown the PET polymer chains into more volatile smaller molecules. This can be achieved by methanolysis. During methanolysis, all the IPA recurring units and TPA recurring units are transformed into respectively dimethylisophthalate and dimethylterephthalate. As a consequence, measuring the molar percentages of dimethylisophthalate is equivalent to measuring the molar percentages of isophthalic acid.

A PET sample (for example 0.3 g) such as a piece of PET bottle or PET fibre can be mixed with NaOH in 5 ml of methanol in a closed reactor. The NaOH can be present 1 % by weight based on the total weight of the NaOH and the methanol.

The PET samples can be obtained through gridding until the PET particles reached sizes between 350 and 430 $\mu$m. In this particular case, the size of the PET particles refers to the greatest dimension of said particle. The mixture can then be heated inside the closed reactor at a temperature of 150°C for at least 30 minutes. The obtained clear solution can then be analysed by HPLC.

General conditions for reversed phase HPLC analysis

The reversed phase HPLC measurements can be carried out on a HPLC apparatus Agilent 1260 series with Agilent G1312B quaternary pump and Agilent G4212BA diode array detector.

Column: Zorbax SB-Phenyl Agilent LC column, 250mm, i.d. 4.6mm, 5$\mu$m or equivalent.
Elution solvent: a mixture of methanol-water (15:85, V/V). The pH of the solvent can be 3.
Flow rate: 1.0 mL/min, solvent temperature: 30°C.
Detector: UV detection at 254 nm wavelength.
Chromatogram recording time: at least 20 min.

HPLC Calibration

The calibration can be carried out using standards which are PET samples with known amounts of dimethylisophthalate, more preferably with 0.9 mol%, 2.3 mol%, 4.7 mol% and 9.4 mol% of dimethylisophthalate. Orthophthalic acid is used internal standard.

The standards can be analysed by HPLC using the same conditions as described in the General conditions for reversed phase HPLC analysis and a calibration curve can be constructed which correlates the peak area ratio with the IPA mole percentage concentration.

Determination of the IPA molar percentage Y of a material M by HPLC

A material M which can be a PET fibre for use in a textile with an unknown IPA molar percentage can be provided.

The material M can be prepared according to the General procedure for samples preparation as defined above.

The mixture obtained after applying the general procedure for samples preparation to the material M can be analysed by HPLC using the general conditions for HPLC analysis as defined above with the above internal standard. The HPLC chromatograph of the mixture resulting from the treatment of material M can be recorded.

Then, the IPA molar percentage Y of said material M can be obtained via the HPLC calibration above.

Determination of the average X of the molar percentages of IPA of the series of

reference materials by HPLC

A series of reference material which contains recycled PET flakes originating from one commercial type of PET beverage bottles belonging to a well-known trademark can be provided.

For each reference material, the molar percentages of IPA can be determined by HPLC using the same determination method which was used above for the determination of the IPA molar percentages of the material M by HPLC.

Then the average X of the IPA molar percentages of said series of reference materials can be calculated.

Determination of the percentage of r-PET in said material M

The percentage of r-PET in said material M is calculated by using the ratio $R_{IPA}$ wherein:

$$R_{IPA} = \frac{Y}{X} \star 100$$

and wherein X is the average of the molar percentages of IPA of the series of reference materials as determined by HPLC and Y is the molar percentage of IPA of said material M as determined by HPLC.

**A method for determining a percentage of recycled polyethylene terephthalate by Near Infrared spectroscopy (NIR)**

General procedure for NIR measurements

The NIR spectroscopy measurement is carried out using a NIR DS2500 Solid Analyzer from Metrohm in reflection mode over the full wavelength range (400-2500 nm) or equivalent. A rotating DS2500 Large Sample Cup was employed. The samples can be analysed without specific preparation. However, if needed, they can be ground.

NIR Calibration

The calibration can be carried out using standards which are PET samples with known amounts of IPA, preferably with 0.9 mol%, 2.3 mol%, 4.7 mol% and 9.4 mol% of IPA.
For each standard prepared, the NIR spectrum can be acquired according to the general procedure for NIR measurement above, including standard baseline correction and multiplicative scatter correction.
The Vision AIR Software (version 8.000.5224) can build a calibration by applying a linear regression method such as a multiple linear least squares regression (MLR). The calibration is carried out considering the whole spectrum of the standards.

Determination of the IPA molar percentage Y of a material M

A material M which can be a PET fibre for use in a textile with an unknown IPA molar percentage can be provided.
The NIR spectrum of said material M can be recorded in the same conditions using the same method and apparatus than for the NIR measurements of the PET standards for the above NIR calibration.
The VisioN AIR Software determines the molar percentages of IPA Y in said material M by using the MLR calibration which was obtained above.

Determination of the average X of the molar percentages of IPA of the series of reference materials

A series of reference material which contains recycled PET flakes originating from one commercial type of PET beverage bottles belonging to a well-known trademark can be provided.
For each reference material, the molar percentages of IPA can be determined by NIR using the same determination method which was used above for the determination of the IPA molar percentages of the material M (i.e. via a MLR calibration using VisioN AIR Software).
Then the average X of the IPA molar percentages of said series of reference materials is calculated.

Determination of the percentage of r-PET in said material M

The percentage of r-PET in said material M is calculated by using the ratio $R_{IPA}$ wherein:

$$R_{IPA} = \frac{Y}{X} \star 100$$

and wherein X is the average of the molar percentages of IPA of the series of reference materials and Y is the molar percentage of IPA of said material M.

**EXAMPLES**

The invention will be now described in more details with reference to the following examples, whose purpose is merely illustrative and not intended to limit the scope of the invention.

*Analysis method*

**$^1$H NMR spectroscopy**

$^1$H NMR spectroscopy was performed using a Bruker 400 MHz NMR spectrometer.

NMR raw spectral data were acquired with the following data acquisition parameters:

- probe temperature: 298 K;
- pulse program: zgig;
- delay D1 $\geq$ 10 s;
- time domain (TD) $\geq$ 32k points;
- sweep width (SW) $\geq$ 15 ppm;
- transmitter frequency offset $^1$H (O1P): 4.0 ppm;
- transmitter frequency offset $^{13}$C (O2P): 75 ppm;
- number of transients (NS) $\geq$ 32.

Sweep width (SW) / time domain (TD) can be adjusted if needed. The resulting spectral window must contain the -0.5 to 10 ppm region.

The resulting Free Induction Decay (FID) was then Fourier transformed after exponential apodization (LB = 0.3 Hz). The respective spectra were phase-corrected and referenced against lock frequency. A general baseline correction was performed and the peak areas of the aromatic resonance signals (*see below*) were integrated in order to calculate the integrals A1 and A2. Bias and slope correction were performed when needed. The integral **A1** was defined as the reference signal with an arbitrary value of 100.0000.

***The determination of the molar ratio of IPA of each reference material of a series of reference materials.***

**Sample preparation:**

Each reference material was concerned with recycled PET flakes originating from one commercial type of PET beverage bottles belonging to a well-known trademark. In total, three different reference materials were used, i.e. Examples 1 - 3 which were respectively concerned with recycled PET flakes from three different commercial types of PET beverage bottles belonging to trademarks 1 - 3, thereby forming a series of reference materials.

$^1$H NMR samples of Examples 1 - 3 were respectively prepared by weighing circa 60.0 mg of the above-mentioned recycled PET flakes in adapted vials. Then, TFA-d$_1$ was added by transferring circa 0.70 mL of individual commercial ampules (0.75 mL) of TFA-d$_1$ to each of said adapted vials. Ampules of TFA-d$_1$ were commercially available and purchased from Eurisotop D022BB. All the mixtures were then vortexed until complete dissolution and homogenization were achieved. These dissolved and homogenized solutions were then transferred to NMR tubes and said NMR tubes were capped with NMR caps.

The prepared $^1$H NMR samples of Examples 1 - 3 were then submitted to the $^1$H NMR spectroscopy analysis method as detailed above. Residual TFA signals were detected as large singlets in the 11 - 12 ppm region. For each of the Examples 1 - 3, the respective $^1$H NMR spectroscopy measurements were repeated five times and for each of said repeated measurements the molar ratio of IPA was calculated according to Formula (II). The molar ratio of IPA for each of said Examples 1 - 3 was then calculated as the averaged value over the five repeated measurements for each of said Examples 1 - 3, respectively. These molar ratios of IPA for each of said Examples 1 - 3, in combination with the standard deviation between the five repeated measurements for each of said Examples 1 - 3, were further summarized in Table 1 below.

Furthermore, as illustrated in Table 1 below, the value X for Examples 1 - 3 , i.e. the average of the molar ratios of IPA for Examples 1 - 3, was calculated.

The molar ratio of IPA in the recycled PET flakes was calculated using the following integrating areas of the aromatic resonance signals in the resulting $^1$H NMR spectrum:

- integral of peak area **A1** from 8.00 to 8.70 ppm:

   ◦ aromatic resonance signal at about 8.3 ppm (broad singlet) corresponding to the four aromatic protons of the recurring units derived from TPA (i.e. aromatic protons at the positions 2, 3, 5, and 6 of the aromatic ring system of said recurring units derived from TPA);

∘ aromatic resonance signal at about 8.5 ppm (doublet) corresponding to the two aromatic protons of the recurring units derived from IPA (i.e. aromatic protons at the positions 4 and 6 of the aromatic ring system of said recurring units derived from IPA);

- integral of peak area **A2** from 7.65 to 7.85 ppm:

∘ aromatic resonance signal at about 7.7 ppm (triplet) corresponding to one aromatic proton of the recurring units derived from IPA (i.e. aromatic proton at the position 5 of the aromatic ring system of said recurring units derived from IPA)

*Table 1:*

|  | **Example 1** | **Example 2** | **Example 3** |
|---|---|---|---|
| molar ratio of IPA (averaged value over 5 repeated measurements) | 2.64 | 2.38 | 2.52 |
| standard deviation (dispersion) | 0.06 | 0.42 | 0.06 |
|  | **Examples 1 - 3** | | |
| X | 2.51 | | |
| standard deviation (dispersion) | 0.25 | | |

Each molar ratio of IPA was measured ten times and showed good repeatability.

In order to further demonstrate the accuracy of the measurements according to the $^1$H NMR spectroscopy analysis method as detailed above, a sweater textile material having the following textile composition (material M) was used, relative to the total weight of said textile composition:

- cotton: 52 wt. %;
- non recycled PET: 31 wt. %; and
- r-PET: 11 wt. %.

The theoretical percentage of r-PET in said sweater is equal to [11 / (11 + 31)] * 100 = 26.2 %.

In particular, Example 4 was a sample originating from the shoulder part of the sweater textile material (material M) having the above textile composition, while Example 5 was a sample originating from the sleeve part of said sweater textile material (material M). Further, two $^1$H NMR samples were prepared of Examples 4 - 5 using TFA-d$_1$ according to the preparation of the $^1$H NMR samples of Examples 1 - 3 above. The prepared $^1$H NMR samples of Examples 4 - 5 were then submitted to the $^1$H NMR spectroscopy analysis method as detailed above.

In order to detect IPA in said $^1$H NMR samples of Examples 4 - 5 at the molar ratio of at least 0.05 %, the signal to noise ratio of the **A1** aromatic resonance signal was ≥ 150.000:1, based on a 200 Hz noise region. If this system suitability test (SST) was not met, the acquisition was repeated with an increased number of transients (NS). For each of the Examples 4 - 5, the respective $^1$H NMR spectroscopy measurements were repeated five times and for each of said repeated measurements the molar ratio of IPA, was calculated according to Formula (II), i.e. in a similar way as compared to the calculation of the molar ratio of IPA according to the Formula (II) as described above for each of the reference materials.

The integrals of aromatic resonance signals **A1** and **A2** have the same meaning as described above. Then, the percentage of r-PET in Examples 4 - 5, i.e. $R_{IPA}$, was calculated according to the Formula I below with previously determined value X equal to 2.51 % ± 0.25 % as derived from the series of reference materials, i.e. recycled PET flakes from three different commercial types of PET beverage bottles belonging to trademarks 1 - 3, as described above (Table 1). Next, $R_{IPA}$ for the Examples 4 - 5 was then compared to its theoretical value of 26.2 %.

$$R_{IPA} = \frac{Y}{X} * 100$$

(Formula I)

The experimental averaged values of $R_{IPA}$ for Examples 4 - 5 were further summarized in Table 2 below.

*Table 2:*

| Example 4 | | | Example 5 | | | |
|---|---|---|---|---|---|---|
| Minimum $R_{IPA}$ (%) | Average $R_{IPA}$ (%) | Maximum $R_{IPA}$ (%) | Minimum $R_{IPA}$ (%) | Average $R_{IPA}$ (%) | Maximum $R_{IPA}$ (%) | **Theoretical $R_{IPA}$ (%)** |
| 16.8 | 21.0 | 25.2 | 17.6 | 22.0 | 26.4 | **26.2** |

As convincingly demonstrated by the results of Examples 4 - 5 as described in Table 2 above, an excellent measurement accuracy was achieved ($\pm$ 4.4 %). The detection limit of IPA in materials having a composition comprising PET and r-PET was 0.10 %, thereby leading to an excellent and high sensitivity of the described $^1$H NMR spectroscopy analysis method. As a direct consequence, the corresponding detection limit of r-PET in said materials was 4.0 %. Since the detection limit of IPA (0.10 %) was significantly smaller, i.e. by a factor of 20, when compared to a typical minimum molar ratio of IPA in recycled PET flakes from PET beverage bottles (circa 2 %), the described $^1$H NMR methodology equally demonstrated a high reliability.

The above methodology was further evaluated with reference to Examples 6 - 9 which were based on the following four different textile materials:

- Example 6: material M is white pillow (outer shell);
- Example 7: material M is white pillow (lining);
- Example 8: material M is a grey fabric; and
- Example 9: material M is a green fabric.

$^1$H NMR samples of Examples 6 - 9 were prepared according to Examples 1 - 5 as described above. The prepared $^1$H NMR samples of Examples 6 - 9 were then submitted to the $^1$H NMR spectroscopy analysis method as detailed above, and the values for $R_{IPA}$ were calculated accordingly.

The experimental averaged values of $R_{IPA}$ for Examples 6 - 9 were further summarized in Table 3 below.

*Table 3:*

| Example 6 | Example 7 | Example 8 | Example 9 |
|---|---|---|---|
| Average $R_{IPA}$ (%) | | | |
| < 0.05 | 50 | < 0.05 | 38 |
| Evaluation of the presence of r-PET in textile materials of Examples 6 - 9 | | | |
| r-PET not present | r-PET partially present | r-PET not present | r-PET partially present |

**Claims**

1. A method for determining a percentage of recycled polyethylene terephthalate [r-PET, hereinafter] in a material [material M, hereinafter] with regards to the total amount of r-PET and non-recycled polyethylene terephthalate comprised in said material M, said r-PET being derived from at least one polymeric material comprising at least one PET polymer and optionally IPA monomers and optionally TPA monomers wherein said at least one PET polymer is derived from recurring units derived from ethylene glycol and recurring units derived from terephthalic acid [TPA, hereinafter] and recurring units derived from isophthalic acid [IPA, hereinafter]; said method comprising the steps of :

    a) determining the molar ratio of IPA or the molar percentages of IPA of each reference material of a series of reference materials, each reference material comprising at least one PET polymer and optionally IPA monomers and optionally TPA monomers, wherein said at least one PET polymer is derived from recurring units derived from ethylene glycol and recurring units derived from TPA and recurring units derived from IPA; wherein the molar ratio of IPA of each reference material of said series of reference materials is the ratio of the molar quantity of the recurring units derived from IPA and when present the IPA monomers to the molar quantity of the recurring units derived from TPA and when present the TPA monomers as comprised in said each reference material of said series of reference materials; and wherein the molar percentage of IPA is the molar percentage of IPA recurring units and when present the IPA monomers as comprised in said each reference material of said series of reference materials;

b) calculating the average of the molar ratios of IPA or of the molar percentages of IPA of the series of reference materials as determined in step a);

c) providing a material M;

d) determining the molar ratio of IPA or the molar percentage of IPA of said material M by analysing said material M using at least one analysis method selected from the group consisting of nuclear magnetic resonance (NMR) spectroscopy, Fourier transform infrared (FTIR) spectroscopy, Raman spectroscopy, gas chromatography (GC), high pressure liquid chromatography (HPLC), Near Infrared spectroscopy (NIR); wherein the molar ratio of IPA of said material M is the ratio of the molar quantity of the recurring units derived from IPA and when present the IPA monomers to the molar quantity of the recurring units derived from TPA and when present the TPA monomers as comprised in said material M; and wherein the molar percentage of IPA of said material M is the molar percentage of IPA recurring units and when present the IPA monomers as comprised in said material M;

e) determining the percentage of r-PET in said material M by calculating the ratio $R_{IPA}$ wherein:

$$R_{IPA} = \frac{Y}{X} * 100$$

and wherein X is the the average of the molar ratios of IPA or of the molar percentages of IPA of the series of reference materials as determined step b) and Y is the molar ratio of IPA or the molar percentage of IPA of said material M as determined in step d) and wherein $X \geq Y$ and X is different from 0.

2. A method according to claim 1, wherein said at least one PET polymer comprised in said at least one polymeric material, comprises at least 80% by weight, preferably at least 85% by weight, more preferably at least 90% by weight, even more preferably at least 95% by weight, by weight of the recurring units derived from TPA and the recurring units derived from ethylene glycol, with regards to the total weight of said at least one PET polymer.

3. A method according to claim 1 or claim 2, wherein said at least one PET polymer comprised in said at least one polymeric material, comprises at least 0.5 % by weight, preferably at least 0.8 % by weight, more preferably at least 1 % by weight, even more preferably at least 1.5 % by weight, by weight of the recurring units derived from IPA, with regards to the total weight of said at least one PET polymer.

4. A method according to any one of claims 1 to 3, wherein said at least one PET polymer comprised in said at least one polymeric material, comprises at most 15 % by weight, preferably at most 10 % by weight, more preferably at most 8 % by weight, even more preferably at most 5 % by weight of the recurring units derived from IPA, with regards to the total weight of said at least one PET polymer.

5. A method according to any one of claims 1 to 4, wherein said molar ratio of IPA or said molar percentage of IPA of each reference material of a series of reference materials is determined by using $^1$H-NMR.

6. A method according to any one of claims 1 to 5, wherein said each reference material comprises at least 90 % by weight, more preferably at least 95 % by weight, even more preferably at least 96 % by weight, even more preferably at least 97% by weight, even more preferably at least 98% by weight, even more preferably at least 99% by weight of said at least one PET polymer, with regards to the total weight of said each reference material.

7. A method according to any one of claims 1 to 6, wherein said at least one PET polymer comprised in said each reference material, comprises at least 80% by weight, preferably at least 85% by weight, more preferably at least 90% by weight, even more preferably at least 95% by weight, by weight of the recurring units derived from TPA and the recurring units derived from ethylene glycol, with regards to the total weight of said at least one PET polymer.

8. A method according to any one of claims 1 to 7, wherein said at least one PET polymer comprised in said each reference material comprises at least 0.5 % by weight, preferably at least 0.8 % by weight, more preferably at least 1 % by weight, even more preferably at least 1.5 % by weight, by weight of the recurring units derived from IPA, with regards to the total weight of said at least one PET polymer.

9. A method according to any one of claims 1 to 8, wherein said molar ratio Y or said molar percentage Y is determined by analysing said material M by using NMR, more preferably $^1$H-NMR, even more preferably liquid state $^1$H-NMR.

10. A method according to any one of claims 1 to 9, where said molar ratios of IPA or said molar percentages of each of the reference material of said series of reference materials is determined by analysing said each of the reference material using NMR, more preferably ¹H-NMR, even more preferably liquid state ¹H-NMR.

**11.** A method according to any one of claims 9 to 10, wherein the step of determining said the molar ratios of IPA of each of the reference material of said series of reference materials by analysing said each reference material of said series of reference materials and/ or the step of determining said molar ratio Y by analysing said material M comprises a step of:

• acquiring at least one ¹H-NMR spectrum of said each reference material of said series of reference materials or said material M,
• calculating in said at least one ¹H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA,
• calculating in said at least one¹H-NMR spectrum, the integral of the triplet peak [hereinafter, A2] corresponding to one aromatic proton of the recurring units derived from IPA, in particular, the triplet peak corresponds to the aromatic proton at the position 5 of the aromatic ring of said recurring units derived from IPA,
• determining said molar ratio Y or the molar ratios of IPA of each of the reference material by applying formula (II):

$$\frac{4 * A2}{(A1 - (2 * A2))}$$

formula (II)

**12.** A method according to any one of claims 9 to 10, wherein, the step of determining said the molar ratios of IPA of each of the reference material of said series of reference materials by analysing said each reference material of said series of reference materials and/ or the step of determining said molar ratio Y by analysing said material M comprises a step of :

• acquiring at least one ¹H-NMR spectrum of said each reference material of said series of reference materials or said material M,
• calculating in said at least one ¹H-NMR spectrum, the integral [hereinafter, A1] of the peak corresponding to the four aromatic protons of the recurring units derived from TPA and the doublet peak corresponding to two aromatic protons of the recurring units derived from IPA, in particular, the doublet peak corresponds to the aromatic protons at the positions 4 and 6 of the aromatic ring of said recurring units derived from IPA;
• calculating in said at least one¹H-NMR spectrum, the integral of the singlet peak [hereinafter, A3] corresponding to one aromatic proton of the recurring units derived from IPA, in particular the singlet peak corresponds to the aromatic proton at the position 2 of the aromatic ring of said recurring units derived from IPA,
• determining said molar ratio Y or the molar ratios of IPA of each of the reference material by applying formula (III):

$$\frac{4 * A3}{(A1 - (2 * A3))}$$

formula (III)

**13.** A method according to any one of claims 9 to 10, wherein the step of determining said the molar ratios of IPA of each of the reference material of said series of reference materials by analysing said each reference material of said series of reference materials and/ or the step of determining said molar ratio Y by analysing said material M comprises a step of :

• acquiring at least one ¹H-NMR spectrum of said each reference material of said series of reference materials or said material M,
• calculating in said at least one ¹H-NMR spectrum, the integral [hereinafter, A1] of the peak corresponding to the four aromatic protons of recurring units derived from TPA and the doublet peak corresponding to two aromatic protons of recurring units derived from IPA, in particular, the doublet peak corresponds to the aromatic protons at the positions 4 and 6 of the aromatic ring of said recurring units derived from IPA,
• calculating in said at least one¹H-NMR spectrum, the integral of the singlet peak [hereinafter, A3] corresponding to one aromatic proton of recurring units derived from IPA, in particular the singlet peak corresponds to the aromatic proton at the position 2 of the aromatic ring of said recurring units derived from IPA,

• calculating in said at least one $^1$H-NMR spectrum, the integral of the triplet peak [hereinafter, A2] corresponding to one aromatic proton of recurring units derived from IPA, in particular the triplet peak corresponds to the aromatic proton at the position 5 of the aromatic ring of said recurring units derived from IPA,

• determining said molar ratio Y or the molar ratios of IPA of each of the reference material by applying formula (IV) or formula (V):

$$\frac{4 * A3}{(A1 - (2 * A2))}$$

Formula (IV)

$$\frac{4 * A2}{(A1 - (2 * A3))}$$

Formula (V)

**14.** A method according to any one of claims 9 to 13, wherein , for determining the molar ratios of IPA or the molar percentages of each of the reference material of said series of reference materials and/or said molar ratio Y or said molar percentage Y, $^1$H-NMR spectroscopy is used with a number of transients (NS) of at least 10, more preferably at least 20, even more preferably at least 30, even more preferably at least 32.

**15.** A method according to any one of claims 9 to 10, wherein the step of determining said the molar percentages of IPA of each of the reference material of said series of reference materials by analysing said each reference material of said series of reference materials and/ or the step of determining said molar percentage Y by analysing said material M comprises a step of:

• acquiring at least one $^1$H-NMR spectrum of said each reference material of said series of reference materials or said material M,

• calculating in said at least one $^1$H-NMR spectrum, the integral [hereinafter, A1] of the peak corresponding to the four aromatic protons of recurring units derived from TPA and the doublet peak corresponding to two aromatic protons of recurring units derived from IPA, in particular, the doublet peak corresponds to the aromatic protons at the positions 4 and 6 of the aromatic ring of said recurring units derived from IPA,

• calculating in said at least one $^1$H-NMR spectrum, the integral of the triplet peak [hereinafter, A2] corresponding to one aromatic proton of recurring units derived from IPA, in particular, the triplet peak corresponds to the aromatic proton at the position 5 of the aromatic ring of said recurring units derived from IPA,

• determining said molar percentage Y or the molar percentages of IPA of each of the reference material by applying formula (VI):

$$\frac{4 * A2}{(A1 - (2 * A2) + (4 * A2))}$$

formula (VI)

**Patentansprüche**

**1.** Verfahren zur Bestimmung des Prozentsatzes an recyceltem Polyethylenterephthalat [r-PET, im Folgenden] in einem Material [Material M, im Folgenden] in Bezug auf die Gesamtmenge an r-PET und nicht recyceltem Polyethylenterephthalat, die in dem Material M umfasst ist, wobei das r-PET aus mindestens einem Polymermaterial abgeleitet ist, das mindestens ein PET-Polymer und optional IPA-Monomere und optional TPA-Monomere umfasst, wobei das mindestens eine PET-Polymer aus wiederkehrenden Einheiten abgeleitet ist, die aus Ethylenglykol abgeleitet sind, und wiederkehrenden Einheiten, die aus Terephthalsäure [TPA, im Folgenden] abgeleitet sind und wiederkehrende Einheiten, die aus Isophthalsäure [IPA, im Folgenden] abgeleitet sind; wobei das Verfahren die Schritte umfasst zum:

a) Bestimmen des Molverhältnisses von IPA oder der Molprozentsätze von IPA jedes Referenzmaterials einer Reihe von Referenzmaterialien, wobei jedes Referenzmaterial mindestens ein PET-Polymer und optional IPA-Monomere und optional TPA-Monomere umfasst, wobei das mindestens eine PET-Polymer aus wiederkehrenden Einheiten abgeleitet ist, die aus Ethylenglykol abgeleitet sind, und wiederkehrenden Einheiten, die aus TPA abgeleitet sind, und wiederkehrenden Einheiten, die aus IPA abgeleitet sind; wobei das Molverhältnis von IPA jedes Referenzmaterials der Reihe von Referenzmaterialien das Verhältnis der Molmenge der wiederkehrenden Einheiten, die aus IPA und, falls vorhanden, den IPA-Monomeren abgeleitet sind, zur Molmenge der wiederkehrenden Einheiten, die aus TPA und, falls vorhanden, den TPA-Monomeren abgeleitet sind, wie in jedem Referenzmaterial der Reihe von Referenzmaterialien umfasst, ist; und wobei der Molprozentsatz von IPA der Molprozentsatz der wiederkehrenden IPA-Einheiten und, falls vorhanden, der IPA-Monomere, wie in jedem Referenzmaterial der Reihe von Referenzmaterialien umfasst, ist;

b) Berechnen des Durchschnitts der Molverhältnisse von IPA oder der Molprozentsätze von IPA der Reihe von Referenzmaterialien, wie in Schritt a) bestimmt;

c) Bereitstellen eines Materials M;

d) Bestimmen des Molverhältnisses von IPA oder des Molprozentsatzes von IPA des Materials M durch Analysieren des Materials M unter Verwendung von mindestens eines Analyseverfahrens, ausgewählt aus der Gruppe bestehend aus Kernspinresonanzspektroskopie (NMR), Fourier-Transform-Infrarotspektroskopie (FTIR), Raman-Spektroskopie, Gaschromatographie (GC), Hochdruckflüssigkeitschromatographie (HPLC), Nahinfrarotspektroskopie (NIR); wobei das Molverhältnis von IPA des Materials M das Verhältnis der Molmenge der wiederkehrenden Einheiten, die aus IPA und, falls vorhanden, den IPA-Monomeren abgeleitet sind, zur Molmenge der wiederkehrenden Einheiten, die aus TPA und, falls vorhanden, den TPA-Monomeren abgeleitet sind, wie in dem Material M umfasst, ist; und wobei der Molprozentsatz von IPA des Materials M der Molprozentsatz von wiederkehrenden IPA-Einheiten und, falls vorhanden, der IPA-Monomere, wie in dem Material M umfasst, ist;

e) Bestimmen des Prozentsatzes an r-PET in dem Material M durch Berechnen des Verhältnisses $R_{IPA}$, wobei:

$$R_{IPA} = \frac{Y}{X} * 100$$

und wobei X der Durchschnitt der Molverhältnisse von IPA oder der Molprozentsätze von IPA der Reihe von Referenzmaterialien, wie in Schritt b) bestimmt, ist, und Y das Molverhältnis von IPA oder der Molprozentsatz von IPA des Materials M, wie in Schritt d) bestimmt, ist, und wobei X≥Y und X ungleich 0 ist.

**2.** Verfahren nach Anspruch 1, wobei das in dem mindestens einen Polymermaterial umfasste mindestens eine PET-Polymer mindestens 80 Gew.-%, vorzugsweise mindestens 85 Gew.-%, bevorzugter mindestens 90 Gew.-%, noch bevorzugter mindestens 95 Gew.-% pro Gewicht der aus TPA abgeleiteten wiederkehrenden Einheiten und der aus Ethylenglykol abgeleiteten wiederkehrenden Einheiten, in Bezug auf das Gesamtgewicht des mindestens einen PET-Polymers, umfasst.

**3.** Verfahren nach Anspruch 1 oder Anspruch 2, wobei das in dem mindestens einen Polymermaterial umfasste mindestens eine PET-Polymer mindestens 0,5 Gew.-%, vorzugsweise mindestens 0,8 Gew.-%, bevorzugter mindestens 1 Gew.-%, noch bevorzugter mindestens 1,5 Gew.-% pro Gewicht der aus IPA abgeleiteten wiederkehrenden Einheiten, in Bezug auf das Gesamtgewicht des mindestens einen PET-Polymers, umfasst.

**4.** Verfahren nach irgendeinem der Ansprüche 1 bis 3, wobei das in dem mindestens einen Polymermaterial umfasste mindestens eine PET-Polymer höchstens 15 Gew.-%, vorzugsweise höchstens 10 Gew.-%, bevorzugter höchstens 8 Gew.-%, noch bevorzugter höchstens 5 Gew.-% der aus IPA abgeleiteten wiederkehrenden Einheiten, in Bezug auf das Gesamtgewicht des mindestens einen PET-Polymers, umfasst.

**5.** Verfahren nach irgendeinem der Ansprüche 1 bis 4, wobei das Molverhältnis von IPA oder der Molprozentsatz von IPA jedes Referenzmaterials einer Reihe von Referenzmaterialien unter Verwendung von $^1$H-NMR bestimmt wird.

**6.** Verfahren nach irgendeinem der Ansprüche 1 bis 5, wobei jedes Referenzmaterial mindestens 90 Gew.-%, vorzugsweise mindestens 95 Gew.-%, noch bevorzugter mindestens 96 Gew.-%, noch bevorzugter mindestens 97 Gew.-%, noch bevorzugter mindestens 98 Gew.-%, noch bevorzugter mindestens 99 Gew.-% des mindestens einen PET-Polymers, in Bezug auf das Gesamtgewicht jedes Referenzmaterials umfasst.

7. Verfahren nach irgendeinem der Ansprüche 1 bis 6, wobei das in jedem Referenzmaterial umfasste mindestens eine PET-Polymer mindestens 80 Gew.-%, vorzugsweise mindestens 85 Gew.-%, bevorzugter mindestens 90 Gew.-%, noch bevorzugter mindestens 95 Gew.-% pro Gewicht der aus TPA abgeleiteten wiederkehrenden Einheiten und der aus Ethylenglykol abgeleiteten wiederkehrenden Einheiten, in Bezug auf das Gesamtgewicht des mindestens einen PET-Polymers, umfasst.

8. Verfahren nach irgendeinem der Ansprüche 1 bis 7, wobei das in jedem Referenzmaterial umfasste mindestens eine PET-Polymer mindestens 0,5 Gew.-%, vorzugsweise mindestens 0,8 Gew.-%, bevorzugter mindestens 1 Gew.-%, noch bevorzugter mindestens 1,5 Gew.-% pro Gewicht der aus IPA abgeleiteten wiederkehrenden Einheiten, in Bezug auf das Gesamtgewicht des mindestens einen PET-Polymers, umfasst.

9. Verfahren nach irgendeinem der Ansprüche 1 bis 8, wobei das Molverhältnis Y oder der Molprozentsatz Y durch Analysieren des Materials M unter Verwendung von NMR, bevorzugter $^1$H-NMR, noch bevorzugter $^1$H-NMR im flüssigen Zustand, bestimmt wird.

10. Verfahren nach irgendeinem der Ansprüche 1 bis 9, wobei die Molverhältnisse von IPA oder die Molprozentsätze jedes Referenzmaterials der Reihe von Referenzmaterialien durch Analysieren jedes Referenzmaterials unter Verwendung von NMR, vorzugsweise $^1$H-NMR, noch bevorzugter $^1$H-NMR im flüssigen Zustand, bestimmt werden.

11. Verfahren nach irgendeinem der Ansprüche 9 bis 10, wobei der Schritt des Bestimmens der Molverhältnisse von IPA jedes Referenzmaterials der Reihe von Referenzmaterialien durch Analysieren jedes Referenzmaterials der Reihe von Referenzmaterialien und/oder der Schritt des Bestimmens des Molverhältnisses Y durch Analysieren des Materials M einen Schritt umfasst zum:

    - Erfassen von mindestens einem $^1$H-NMR-Spektrum jedes Referenzmaterials der Serie von Referenzmaterialien oder des Materials M,
    - Berechnen in dem mindestens einen $^1$H-NMR-Spektrum des Integrals [im Folgenden, A1] der Spitze, die den vier aromatischen Protonen der aus TPA abgeleiteten wiederkehrenden Einheiten entspricht, und der Dublettspitze, die den beiden aromatischen Protonen der aus IPA abgeleiteten wiederkehrenden Einheiten entspricht, wobei insbesondere die Dublettspitze den aromatischen Protonen an den Positionen 4 und 6 des aromatischen Rings der aus IPA abgeleiteten wiederkehrenden Einheiten entspricht,
    - Berechnen in dem mindestens einen $^1$H-NMR-Spektrum des Integrals der Triplettspitze [im Folgenden, A2], die einem aromatischen Proton der aus IPA abgeleiteten wiederkehrenden Einheiten entspricht, wobei insbesondere die Triplettspitze dem aromatischen Proton an Position 5 des aromatischen Rings der aus IPA abgeleiteten wiederkehrenden Einheiten entspricht,
    - Bestimmen des Molverhältnisses Y oder der Molverhältnisse von IPA jedes Referenzmaterials durch Anwenden der Formel (II):

$$\frac{4*A2}{(A1-(2*A2))}$$

Formel (II)

12. Verfahren nach irgendeinem der Ansprüche 9 bis 10, wobei der Schritt des Bestimmens der Molverhältnisse von IPA jedes Referenzmaterials der Reihe von Referenzmaterialien durch Analysieren jedes Referenzmaterials der Reihe von Referenzmaterialien und/oder der Schritt des Bestimmens des Molverhältnisses Y durch Analysieren des Materials M einen Schritt umfasst zum:

    - Erfassen von mindestens einem $^1$H-NMR-Spektrum jedes Referenzmaterials der Serie von Referenzmaterialien oder des Materials M,
    - Berechnen in dem mindestens einen $^1$H-NMR-Spektrum des Integrals [im Folgenden, A1] der Spitze, die den vier aromatischen Protonen der aus TPA abgeleiteten wiederkehrenden Einheiten entspricht, und der Dublettspitze, die den beiden aromatischen Protonen der aus IPA abgeleiteten wiederkehrenden Einheiten entspricht, wobei insbesondere die Dublettspitze den aromatischen Protonen an den Positionen 4 und 6 des aromatischen Rings der aus IPA abgeleiteten wiederkehrenden Einheiten entspricht;
    - Berechnen in dem mindestens einen $^1$H-NMR-Spektrum des Integrals der Singulettspitze [im Folgenden, A3], die einem aromatischen Proton der aus IPA abgeleiteten wiederkehrenden Einheiten entspricht, wobei insbesondere die Singulettspitze dem aromatischen Proton an Position 2 des aromatischen Rings der aus IPA abgeleiteten wiederkehrenden Einheiten entspricht,
- Bestimmen des Molverhältnisses Y oder der Molverhältnisse von IPA jedes Referenzmaterials durch Anwenden der Formel (III):

$$\frac{4*A3}{(A1-(2*A3))}$$

Formel (III)

13. Verfahren nach irgendeinem der Ansprüche 9 bis 10, wobei der Schritt des Bestimmens der Molverhältnisse von IPA jedes Referenzmaterials der Reihe von Referenzmaterialien durch Analysieren jedes Referenzmaterials der Reihe von Referenzmaterialien und/oder der Schritt des Bestimmens des Molverhältnisses Y durch Analysieren des Materials M einen Schritt umfasst zum:

    - Erfassen von mindestens einem $^1$H-NMR-Spektrum jedes Referenzmaterials der Serie von Referenzmaterialien oder des Materials M,
    - Berechnen in dem mindestens einen $^1$H-NMR-Spektrum des Integrals [im Folgenden, A1] der Spitze, die den vier aromatischen Protonen von aus TPA abgeleiteten wiederkehrenden Einheiten entspricht, und der Dublettspitze, die den beiden aromatischen Protonen von aus IPA abgeleiteten wiederkehrenden Einheiten entspricht, wobei insbesondere die Dublettspitze den aromatischen Protonen an den Positionen 4 und 6 des aromatischen Rings der aus IPA abgeleiteten wiederkehrenden Einheiten entspricht,
    - Berechnen in dem mindestens einen $^1$H-NMR-Spektrum des Integrals der Singulettspitze [im Folgenden, A3], die einem aromatischen Proton von aus IPA abgeleiteten wiederkehrenden Einheiten entspricht, wobei insbesondere die Singulettspitze dem aromatischen Proton an Position 2 des aromatischen Rings der aus IPA abgeleiteten wiederkehrenden Einheiten entspricht,
    - Berechnen in dem mindestens einen $^1$H-NMR-Spektrum des Integrals der Triplettspitze [im Folgenden, A2], die einem aromatischen Proton von aus IPA abgeleiteten wiederkehrenden Einheiten entspricht, wobei insbesondere die Triplettspitze dem aromatischen Proton an Position 5 des aromatischen Rings der aus IPA abgeleiteten wiederkehrenden Einheiten entspricht,
    - Bestimmen des Molverhältnisses Y oder der Molverhältnisse von IPA jedes Referenzmaterials durch Anwenden der Formel (IV) oder der Formel (V):

$$\frac{4*A3}{(A1-(2*A2))}$$

Formel (IV)

$$\frac{4*A2}{(A1-(2*A3))}$$

Formel (V)

14. Verfahren nach irgendeinem der Ansprüche 9 bis 13, wobei zur Bestimmung der Molverhältnisse von IPA oder der Molprozentsätze jedes Referenzmaterials der Reihe von Referenzmaterialien und/oder des Molverhältnisses Y oder des Molprozentsatzes Y $^1$H-NMR-Spektroskopie mit einer Anzahl von Transienten (NS) von mindestens 10, bevorzugter mindestens 20, noch bevorzugter mindestens 30, noch bevorzugter mindestens 32 verwendet wird.

15. Verfahren nach irgendeinem der Ansprüche 9 bis 10, wobei der Schritt des Bestimmens der Molprozentsätze von IPA jedes Referenzmaterials der Reihe von Referenzmaterialien durch Analysieren jedes Referenzmaterials der Reihe von Referenzmaterialien und/oder der Schritt des Bestimmens des Molprozentsatzes Y durch Analysieren des Materials M einen Schritt umfasst zum:

    - Erfassen von mindestens einem $^1$H-NMR-Spektrum jedes Referenzmaterials der Serie von Referenzmaterialien oder des Materials M,
    - Berechnen in dem mindestens einen $^1$H-NMR-Spektrum des Integrals [im Folgenden, A1] der Spitze, die den vier aromatischen Protonen von aus TPA abgeleiteten wiederkehrenden Einheiten entspricht, und der Dublettspitze, die den beiden aromatischen Protonen von aus IPA abgeleiteten wiederkehrenden Einheiten entspricht, wobei insbesondere die Dublettspitze den aromatischen Protonen an den Positionen 4 und 6 des aromatischen Rings der aus IPA abgeleiteten wiederkehrenden Einheiten entspricht,
- Berechnen in dem mindestens einen $^1$H-NMR-Spektrum des Integrals der Triplettspitze [im Folgenden, A2], die einem aromatischen Proton von aus IPA abgeleiteten wiederkehrenden Einheiten entspricht, wobei insbesondere die Triplettspitze dem aromatischen Proton an Position 5 des aromatischen Rings der aus IPA abgeleiteten wiederkehrenden Einheiten entspricht,
- Bestimmen des Molprozentsatzes Y oder der Molprozentsätze von IPA jedes Referenzmaterials durch Anwenden der Formel (VI):

$$\frac{4*A2}{(A1-(2*A2)+(4*A2))}$$

Formel (VI)

**Revendications**

1. Procédé de détermination d'un pourcentage de polyéthylène téréphtalate recyclé [r-PET, ci-après] dans un matériau [matériau M, ci-après] par rapport à la quantité totale de r-PET et de polyéthylène téréphtalate non recyclé compris dans ledit matériau M, ledit r-PET étant dérivé d'au moins un matériau polymère comprenant au moins un polymère PET et éventuellement des monomères IPA et éventuellement des monomères TPA, dans lequel ledit au moins un polymère PET est dérivé d'unités récurrentes dérivées de l'éthylène glycol et d'unités récurrentes dérivées de l'acide téréphtalique [TPA, ci-après] et d'unités récurrentes dérivées de l'acide isophtalique [IPA, ci-après] ; ledit procédé comprenant les étapes consistant à :

   a) déterminer le rapport molaire de l'IPA ou les pourcentages molaires de l'IPA de chaque matériau de référence d'une série de matériaux de référence, chaque matériau de référence comprenant au moins un polymère PET et éventuellement des monomères IPA et éventuellement des monomères TPA, dans lequel ledit au moins un polymère PET est dérivé d'unités récurrentes dérivées de l'éthylène glycol et d'unités récurrentes dérivées du TPA et d'unités récurrentes dérivées de l'IPA ; dans lequel le rapport molaire de l'IPA de chaque matériau de référence de ladite série de matériaux de référence est le rapport de la quantité molaire des unités récurrentes dérivées de l'IPA et lorsque les monomères IPA sont présents à la quantité molaire des unités récurrentes dérivées du TPA et lorsque les monomères TPA sont présents, tels que compris dans ledit chaque matériau de référence de ladite série de matériaux de référence ; et dans lequel le pourcentage molaire de l'IPA est le pourcentage molaire des unités récurrentes de l'IPA et lorsque les monomères IPA sont présents, tels que compris dans ledit chaque matériau de référence de ladite série de matériaux de référence ;
   b) calculer la moyenne des rapports molaires de IPA ou des pourcentages molaires de IPA de la série de matériaux de référence déterminés à l'étape a) ;
   c) fournir un matériau M ;
   d) déterminer le rapport molaire de l'IPA ou le pourcentage molaire d'IPA dudit matériau M en analysant ledit matériau M en utilisant au moins un procédé d'analyse choisie dans le groupe constitué de la spectroscopie de résonance magnétique nucléaire (RMN), la spectroscopie infrarouge à transformée de Fourier (FTIR), la spectroscopie Raman, la chromatographie en phase gazeuse (GC), la chromatographie liquide à haute pression (HPLC), la spectroscopie dans le proche infrarouge (NIR) ; dans lequel le rapport molaire de l'IPA dudit matériau M est le rapport de la quantité molaire des unités récurrentes dérivées de l'IPA et lorsque les monomères IPA sont présents à la quantité molaire des unités récurrentes dérivées du TPA et lorsque les monomères TPA sont présents dans ledit matériau M ; et dans lequel le pourcentage molaire de l'IPA dudit matériau M est le pourcentage molaire des unités récurrentes de l'IPA et lorsque les monomères IPA sont présents dans ledit matériau M ;
   e) déterminer le pourcentage de r-PET dans ledit matériau M en calculant le rapport $R_{IPA}$ dans lequel :

$$R_{IPA} = \frac{Y}{X}*100$$

   et dans lequel X est la moyenne des rapports molaires de l'IPA ou des pourcentages molaires de l'IPA de la série de matériaux de référence tels que déterminés à l'étape b) et Y est le rapport molaire de l'IPA ou le pourcentage molaire de l'IPA dudit matériau M tel que déterminé à l'étape d) et dans lequel X≥Y et X est différent de 0.

2. Procédé selon la revendication 1, dans lequel ledit au moins un polymère PET compris dans ledit au moins un matériau polymère, comprend au moins 80 % en poids, de préférence au moins 85 % en poids, plus préférentiellement au moins 90 % en poids, encore plus préférentiellement au moins 95 % en poids, en poids des unités récurrentes dérivées du TPA et des unités récurrentes dérivées de l'éthylène glycol, par rapport au poids total dudit au moins un polymère PET.

3. Procédé selon la revendication 1 ou la revendication 2, dans lequel ledit au moins un polymère PET compris dans ledit au moins un matériau polymère comprend au moins 0,5 % en poids, de préférence au moins 0,8 % en poids, de préférence au moins 1 % en poids, de préférence encore au moins 1,5 % en poids, en poids d'unités récurrentes dérivées de l'IPA, par rapport au poids total dudit au moins un polymère PET.

4. Procédé selon l'une des revendications 1 à 3, dans lequel ledit au moins un polymère PET compris dans ledit au moins un matériau polymère comprend au maximum 15 % en poids, de préférence au maximum 10 % en poids, de préférence au maximum 8 % en poids, de préférence encore au maximum 5 % en poids des unités récurrentes dérivées de l'IPA, par rapport au poids total dudit au moins un polymère PET.

5. Procédé selon l'une quelconque des revendications 1 à 4, dans lequel ledit rapport molaire de l'IPA ou ledit pourcentage molaire de l'IPA de chaque matériau de référence d'une série de matériaux de référence est déterminé en utilisant la RMN$^1$H.

6. Procédé selon l'une quelconque des revendications 1 à 5, dans lequel chaque matériau de référence comprend au moins 90 % en poids, de préférence au moins 95 % en poids, de préférence encore au moins 96 % en poids, de préférence encore au moins 97 % en poids, de préférence encore au moins 98 % en poids, de préférence encore au moins 99 % en poids dudit au moins un polymère PET, par rapport au poids total dudit matériau de référence.

7. Procédé selon l'une quelconque des revendications 1 à 6, dans lequel ledit au moins un polymère PET compris dans ledit matériau de référence comprend au moins 80 % en poids, de préférence au moins 85 % en poids, plus préférentiellement au moins 90 % en poids, encore plus préférentiellement au moins 95 % en poids, en poids des unités récurrentes dérivées du TPA et des unités récurrentes dérivées de l'éthylène glycol, par rapport au poids total dudit au moins un polymère PET.

8. Procédé selon l'une quelconque des revendications 1 à 7, dans lequel ledit au moins un polymère PET compris dans ledit matériau de référence comprend au moins 0,5 % en poids, de préférence au moins 0,8 % en poids, de préférence au moins 1 % en poids, de préférence encore au moins 1,5 % en poids, en poids d'unités récurrentes dérivées de l'IPA, par rapport au poids total dudit au moins un polymère PET.

9. Procédé selon l'une quelconque des revendications 1 à 8, dans lequel le rapport molaire Y ou ledit pourcentage molaire Y est déterminé en analysant ledit matériau M par RMN, de préférence par RMN$^1$H, de préférence encore par RMN$^1$H à l'état liquide.

10. Procédé selon l'une quelconque des revendications 1 à 9, dans lequel lesdits rapports molaires de l'IPA ou lesdits pourcentages molaires de chacun des matériaux de référence de ladite série de matériaux de référence sont déterminés en analysant ledit matériau de référence en utilisant la RMN, plus préférentiellement la RMN$^1$H, encore plus préférentiellement la RMN$^1$H à l'état liquide.

11. Procédé selon l'une quelconque des revendications 9 à 10, dans lequel l'étape de détermination desdits rapports molaires de l'IPA de chacun des matériaux de référence de ladite série de matériaux de référence en analysant ledit chaque matériau de référence de ladite série de matériaux de référence et/ou l'étape de détermination dudit rapport molaire Y en analysant ledit matériau M comprend une étape consistant à

• acquérir au moins un spectre RMN$^1$H dudit matériau de référence de ladite série de matériaux de référence ou dudit matériau M,
• calculer, dans ledit au moins un spectre RMN$^1$H, l'intégrale [ci-après, A1] du pic correspondant aux quatre protons aromatiques des unités récurrentes dérivées du TPA et du doublet de pics correspondant aux deux protons aromatiques des unités récurrentes dérivées de l'IPA, en particulier, le doublet de pics correspond aux protons aromatiques aux positions 4 et 6 du cycle aromatique desdites unités récurrentes dérivées de l'IPA,
• calculer dans ledit au moins un spectre RMN$^1$H, l'intégrale du pic triplet [ci-après, A2] correspondant à un proton aromatique des unités récurrentes dérivées de l'IPA, en particulier, le pic triplet correspond au proton aromatique à la position 5 du cycle aromatique desdites unités récurrentes dérivées de l'IPA,
• déterminer ledit rapport molaire Y ou les rapports molaires de l'IPA de chacun des matériaux de référence en appliquant la formule (II) :

$$\frac{4 * A2}{(A1 - (2 * A2))}$$

formule (II)

**12.** Procédé selon l'une quelconque des revendications 9 à 10, dans lequel, l'étape de détermination desdits rapports molaires de l'IPA de chacun des matériaux de référence de ladite série de matériaux de référence en analysant ledit chaque matériau de référence de ladite série de matériaux de référence et/ou l'étape de détermination dudit rapport molaire Y en analysant ledit matériau M comprend une étape consistant à :

• acquérir au moins un spectre RMN$^1$H dudit matériau de référence de ladite série de matériaux de référence ou dudit matériau M,
• calculer, dans ledit au moins un spectre RMN$^1$H, l'intégrale [ci-après, A1] du pic correspondant aux quatre protons aromatiques des unités récurrentes dérivées du TPA et du doublet de pics correspondant aux deux protons aromatiques des unités récurrentes dérivées de l'IPA, en particulier, le doublet de pics correspond aux protons aromatiques aux positions 4 et 6 du cycle aromatique desdites unités récurrentes dérivées de l'IPA ;
• calculer dans ledit au moins un spectre RMN$^1$H, l'intégrale du pic singulet [ci-après, A3] correspondant à un proton aromatique des unités récurrentes dérivées de l'IPA, en particulier le pic singulet correspondant au proton aromatique en position 2 du cycle aromatique desdites unités récurrentes dérivées de l'IPA,
• déterminer ledit rapport molaire Y ou les rapports molaires de l'IPA de chacun des matériaux de référence en appliquant la formule (III) :

$$\frac{4 * A3}{(A1 - (2 * A3))}$$

formule (III)

**13.** Procédé selon l'une quelconque des revendications 9 à 10, dans lequel l'étape de détermination desdits rapports molaires de l'IPA de chacun des matériaux de référence de ladite série de matériaux de référence en analysant ledit chaque matériau de référence de ladite série de matériaux de référence et/ou l'étape de détermination dudit rapport molaire Y en analysant ledit matériau M comprend une étape consistant à :

• acquérir au moins un spectre RMN$^1$H dudit matériau de référence de ladite série de matériaux de référence ou dudit matériau M,
• calculer, dans ledit au moins un spectre RMN$^1$H, l'intégrale [ci-après, A1] du pic correspondant aux quatre protons aromatiques des unités récurrentes dérivées du TPA et du doublet de pics correspondant aux deux protons aromatiques des unités récurrentes dérivées de l'IPA, en particulier, le doublet de pics correspond aux protons aromatiques aux positions 4 et 6 du cycle aromatique desdites unités récurrentes dérivées de l'IPA,
• calculer, dans ledit au moins un spectre RMN$^1$H, l'intégrale du pic singulet [ci-après, A3] correspondant à un proton aromatique des unités récurrentes dérivées de l'IPA, en particulier le pic singulet correspondant au proton aromatique en position 2 du cycle aromatique desdites unités récurrentes dérivées de l'IPA,
• calculer, dans ledit au moins un spectre RMN$^1$H, l'intégrale du pic triplet [ci-après, A2] correspondant à un proton aromatique des unités récurrentes dérivées de l'IPA, en particulier le pic triplet correspondant au proton aromatique en position 5 du cycle aromatique desdites unités récurrentes dérivées de l'IPA,
• déterminer ledit rapport molaire Y ou les rapports molaires de l'IPA de chacun des matériaux de référence en appliquant la formule (IV) ou la formule (V) :

$$\frac{4 * A3}{(A1 - (2 * A2))}$$

Formule (IV)

$$\frac{4 * A2}{(A1 - (2 * A3))}$$

Formule (V)

**14.** Procédé selon l'une quelconque des revendications 9 à 13, dans lequel, pour déterminer les rapports molaires de l'IPA ou les pourcentages molaires de chacun des matériaux de référence de ladite série de matériaux de référence et/ou ledit rapport molaire Y ou ledit pourcentage molaire Y, on utilise la spectroscopie RMN[1]H avec un nombre de transitoires (NS) d'au moins 10, plus préférentiellement d'au moins 20, encore plus préférentiellement d'au moins 30, encore plus préférentiellement d'au moins 32.

**15.** Procédé selon l'une quelconque des revendications 9 à 10, dans lequel l'étape de détermination desdits pourcentages molaires de l'IPA de chacun des matériaux de référence de ladite série de matériaux de référence en analysant ledit chaque matériau de référence de ladite série de matériaux de référence et/ou l'étape de détermination dudit pourcentage molaire Y en analysant ledit matériau M comprend une étape consistant à

• acquérir au moins un spectre RMN[1]H dudit matériau de référence de ladite série de matériaux de référence ou dudit matériau M, calculer, dans ledit au moins un spectre RMN[1]H, l'intégrale [ci-après, A1] du pic correspondant aux quatre protons aromatiques des unités récurrentes dérivées du TPA et du doublet de pics correspondant aux deux protons aromatiques des unités récurrentes dérivées de l'IPA, en particulier, le doublet de pics correspond aux protons aromatiques aux positions 4 et 6 du cycle aromatique desdites unités récurrentes dérivées de l'IPA,
• calculer, dans ledit au moins un spectre RMN[1]H, l'intégrale du pic triplet [ci-après, A2] correspondant à un proton aromatique des unités récurrentes dérivées de l'IPA, en particulier, le pic triplet correspond au proton aromatique en position 5 du cycle aromatique desdites unités récurrentes dérivées de l'IPA ;
• déterminer ledit pourcentage molaire Y ou les pourcentages molaires de l'IPA de chacun des matériaux de référence en appliquant la formule (VI) :

$$\frac{4 * A2}{(A1 - (2 * A2) + (4 * A2))}$$

formule (VI)

*Fig. 1*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8063374 B2

**Non-patent literature cited in the description**

- **YÜKSEKKAYA et al.** *Journal of Engineered Fibers and Fabrics*, 2016, vol. 11, 68-76
- **TELLI et al.** *Journal of Engineered Fibers and Fabrics*, 2015, vol. 10, 47-60
- **MARI et al.** *Journal of Textile Engineering*, 2004, vol. 50, 25-30
- *MicroLab Quant for quantitative method development*, 2019